# EUROPÄISCHE PATENTSCHRIFT

(11) **EP 2 023 771 B1**
(45) Veröffentlichungstag und Bekanntmachung des Hinweises auf die Patenterteilung: **14.12.2016**
(21) Anmeldenummer: 07725790.5
(22) Anmeldetag: 04.06.2007
(51) Int. Cl.: A46B 9/04

(54) **MUNDHYGIENEGERÄT**
ORAL HYGIENE DEVICE
APPAREIL POUR L' HYGIENE BUCCALE

(30) Priorität: 02.06.2006 DE 102006025825
(43) Veröffentlichungstag der Anmeldung: 18.02.2009
(73) Patentinhaber: Braun GmbH, 61476 Kronberg im Taunus (DE)
(72) Erfinder: ZIMMERMANN, Lucy, 61476 Kronberg/Taunus (DE); ANSARI, Petra, 65779 Kelkheim (DE); KLING, Björn, 61479 Glashütten (DE); BIRK, Andreas, 61476 Kronberg/Taunus (DE); WASOW, Sören, 63579 Freigericht (DE)
(86) Internationale Anmeldenummer: PCT/EP2007/004921
(87) Internationale Veröffentlichungsnummer: WO 2007/140959

(56) Entgegenhaltungen:
- WO-A-01/45573
- WO-A-2006/055369
- DE-U1-202005 009 026
- GB-A- 2 391 462
- US-A1- 2004 255 416

## Beschreibung

Die Erfindung betrifft ein Mundhygienegerät, welches insbesondere zum Entfernen von Schmutzpartikeln und Ablagerungen aus dem Innenraum der Mundhöhle ausgebildet ist. Hierzu ist ein in die Mundhöhle einführbares Kopfteil mit einer im Wesentlichen ebenen Grundfläche vorgesehen, an welcher vorspringende Reinigungselemente angeordnet sind.

Mundhygienegeräte sind als solche im Stand der Technik, so zum Beispiel in Form einer mit einem Zungenreiniger kombinierten Zahnbürste aus der US 2006/0026784 A1 bekannt. An einer den Zahnbürstenborsten abgewandten rückseitigen Fläche des Zahnbürstenkopfs sind mehrere vorspringende Elemente, wie etwa länglich ausgebildete Zungenschaberelemente, aber auch einzelne diskrete noppenförmige Vorsprünge angeordnet.

Die quer über die Rückseite des Bürstenkopfes verlaufenden Zungenschaberelemente sind hierbei aus einem relativ hartem Material, wie zum Beispiel Polypropylen gefertigt, während die diskreten Vorsprünge vorzugsweise aus einem relativ weichen Material, wie etwa einem thermoplastischen Elastomer bestehen.

Die länglich ausgebildeten und aus einem harten Material bestehenden Zungenschaberelemente können hinsichtlich ihrer Seitenwände unterschiedliche Steigungen gegenüber der Grundfläche aufweisen, um somit ein aggressiveres Schaberverhalten zur Verfügung zu stellen, wenn das Kopfteil des Mundhygienegeräts vom Benutzer entlang der Zungenoberfläche bewegt wird.

Aus der DE 202005009026 U1 ist ein Mundhygienegerät nach den Merkmalen des Oberbegriffs von Anspruch 1 bekannt.

Eine Zungenoberfläche weist typischerweise eine Vielfalt unterschiedlicher Erhöhungen und Vertiefungen auf, wie etwa Fadenpapillen, Pilzpapillen, Blätterpapillen oder Wallpapillen. Diese unterschiedlich ausgebildeten Papillen mit unterschiedlicher Geometrie sind zudem nicht homogen, sondern sind bevorzugt in unterschiedlichen Flächenabschnitten an der Zungenoberfläche angeordnet.

Dieser komplexen und irregulären Beschaffenheit der zu reinigenden Zungenoberfläche tragen die aus dem Stand der Technik bekannten Reinigungsstrukturen zumeist nicht genügend Rechnung. Weiterhin ist die Struktur und die Formgebung der Reinigungselemente nur auf die Entfernung von Zungenbelag und Ablagerungen von der zu reinigenden Oberfläche ausgelegt.

Solche Reinigungsstrukturen, die über ein gutes Reinigungsverhalten verfügen weisen zumeist eine hohe Dichte einzelner Reinigungselemente und Vorsprünge auf, die aber nur sehr schwer und unzureichend nach der Benutzung des Mundhygienegeräts zu reinigen sind. Dem Reinigungsaspekt des Mundhygienegeräts selbst wird daher nicht genügend Rechnung getragen. Dies ist jedoch gerade bei wiederholter Benutzung des Geräts von großer Bedeutung. Denn gerade solche nach einem Reinigungsvorgang an dem Gerät anhaftenden und zumeist bakteriellen Ablagerungen können eine Quelle für weitere Infektionen sein. Andernfalls kann gerade die wiederholte oder regelmäßige Verwendung des Mundhygienegeräts zu Verschmutzungen und zur Ausbreitung von Bakterien und Keimen innerhalb der Mundhöhle führen.

### Aufgabe

Der vorliegenden Erfindung liegt demgegenüber die Aufgabe zugrunde, ein insbesondere für die Zungenreinigung vorgesehenes Mundhygienegerät zur Verfügung zu stellen, welches sowohl hinsichtlich einer schonenden und effektiven Aufnahme von Schmutzpartikeln einerseits und für ein einfaches und schnelles Reinigen von entfernten Schmutzpartikeln und Ablagerungen andererseits verbessert ist.

So soll mit der Erfindung ein Zungenreiniger zur Verfügung gestellt werden, der nach Anwendung selbst leichter von aufgenommenen Schmutzpartikeln, Bakterien, Keimen und dergleichen zu reinigen ist.

### Erfindung und vorteilhafte Wirkungen

Die Erfindung betrifft ein Mundhygienegerät mit einem Griffteil und einem Kopfteil, an welchem eine im Wesentlichen ebene Grundfläche vorgesehen ist. An dieser Grundfläche sind mehrere, im Wesentlichen senkrecht, zumindest aber schräg zur Grundfläche vorspringende Reinigungselemente vorgesehen, die zumindest zwei unterschiedliche Grundgeometrien aufweisen.

Zumindest einige dieser Reinigungselemente sind als längliche Reinigungsstäbchen ausgebildet, die sich in der Ebene parallel zur Grundfläche erstrecken. Weiterhin ist zumindest ein quer zur Längsrichtung von Reinigungsstäbchen verlaufender und aus Zwischenräumen zwischen unmittelbar benachbart angeordneten Reinigungselementen gebildeter Kanal vorgesehen. Dieser dient zum Transport von Ablagerungen, die vom Mundhygienegerät von der zu reinigenden Gewebeoberfläche aufgenommen wurden. Gleichzeitig ermöglicht ein solcher, quer zu den Reinigungsstäbchen verlaufender Kanal einer einfachen und effektiveren Reinigung des Mundhygienegeräts nach Gebrauch.

Die Ausbildung zumindest eines quer zu den Reinigungsstäbchen verlaufenden Kanals ist insbesondere für eine gleichmäßige Verteilung von Ablagerungen über die Grundfläche des Mundhygienegeräts von Vorteil und ermöglicht somit eine effektivere Ausnutzung der gesamten Reinigungsfläche. Dadurch, dass die Reinigungsstäbchen von dem zumindest einem Kanal in Querrichtung durchzogen sind, können sich die vom Mundhygienegerät aufgenommerien Schmutzpartikel und Ablagerungen nicht nur entlang, sondern auch quer zu den Reinigungsstäbchen verteilen.

Dies ist insbesondere bei der Anwendung des Mundhygienegeräts von Vorteil, wenn sich die zu entfernenden Ablagerungen und Schmutzpartikel z.B. aufgrund der komplexen Beschaffenheit der Zungenoberfläche inhomogen und unterschiedlich an verschiedenen Flächenabschnitten der Grundfläche anlagern. So können im Gebrauch einige in einem bestimmten Flächenabschnitt des Mundhygienegeräts liegende Zwischenräume zwischen Reinigungselementen bereits vollständig mit Ablagerungen oder Schmutzpartikeln zugesetzt sein, wodurch ihre Reinigungswirkung negativ beeinträchtigt wird. Während hingegen an Reinigungselementen eines anderen Flächenabschnitts eine weniger starke Ablagerung stattfindet.

Betrachtet man insbesondere, dass Mundhygienegeräte mit an der Rückseite eines Zahnbürstenkopfes angeordneten Reinigungselementen vom Anwender bevorzugt entlang einer einzigen Bewegungsrichtung über die Zungenoberfläche bewegt werden, so lagern sich die vom Zungenreiniger aufgenommenen Schmutzpartikel z.B. bevorzugt an einem sich unmittelbar an das Griffteil anschließenden Flächenabschnitt des Kopfteils an.

Der erfindungsgemäße und schräg zu den länglichen Reinigungsstäbchen verlaufende Kanal ermöglicht demgegenüber eine gleichmäßige Verteilung von Schmutzpartikeln über der gesamten Reinigungsfläche des Mundhygienegeräts einerseits und erleichtert zudem ein Abwaschen und Reinigen des Mundhygienegeräts nach Anwendung andererseits. Somit kann ein über die gesamte Grundfläche möglichst gleichmäßiger Grad an Ablagerungen erreicht werden, sodass die Funktionalität einzelner Reinigungselemente weniger beeinträchtigt wird.

Als Reinigungselemente unterschiedlicher Grundgeometrien sind zumindest länglich ausgebildete Reinigungsstäbchen und noppenartig ausgebildete Reinigungsnoppen vorgesehen. Während die Reinigungsstäbchen einen im Wesentlichen geradlinigen länglichen Verlauf entlang der Grundfläche aufweisen, haben die Reinigungsnoppen eine demgegenüber geringere laterale Ausdehnung in der Ebene der Grundfläche. Diese laterale Ausdehnung der Noppen in der Ebene der Grundfläche entspricht typischerweise im Wesentlichen der Breite der Reinigungsstäbchen.

Entsprechend ihrer Geometrie erfüllen die Reinigungsstäbchen und die Reinigungsnoppen unterschiedliche Funktionen bei der Reinigung der Zungenoberfläche. So ist insbesondere vorgesehen, dass die Reinigungsnoppen zum Loslösen von an der Zunge anhaftenden Ablagerungen und Schmutzpartikeln, wie etwa Bakterien, Speiseresten oder dergleichen ausgebildet sind.

Die Reinigungsstäbchen sind demgegenüber derart ausgebildet, dass sie die von den Reinigungsnoppen losgelösten Schmutzpartikel und Ablagerungen entlang einer Reinigungsrichtung bewegen und verschieben. Sie sind daher vornehmlich für den Abtransport von losgelösten Schmutzpartikeln und Ablagerungen aus der Mundhöhle ausgebildet.

In der Verlängerung der Längserstreckung von Reinigungsstäbchen ist zumindest eine Reinigungsnoppe angeordnet.

Nach einer weiteren Ausführungsform ist vorgesehen, dass der zumindest eine Kanal im Wesentlichen senkrecht zur Längsrichtung der jeweiligen an den Kanal angrenzenden Reinigungsstäbchen verläuft. Eine derartige Anordnung ist insbesondere dann von Vorteil, wenn die Reinigungsstäbchen mit ihrer Längsrichtung senkrecht zur Bewegungsrichtung des Mundhygienegeräts angeordnet sind, sodass sich die von den Reinigungsstäbchen transportierten Ablagerungen und Schmutzpartikel durch den zumindest einen Kanal über der Grundfläche verteilen können. Auf diese Art und Weise wird einem vollständigen Zusetzen der von den Reinigungsstäbchen gebildeten Erhöhungen mit Schmutzpartikeln effektiv entgegengewirkt.

Weiterhin ist vorgesehen, dass zumindest zwei in Zwischenräumen von unmittelbar benachbart angeordneten Reinigungselementen verlaufende Kanäle vorgesehen sind, die zumindest bereichsweise schräg zueinander verlaufen. Die Realisierung mehrerer und bereichsweise schräg zueinander verlaufender Kanäle ist insbesondere für eine homogene Verteilung von Schmutzpartikeln über die gesamte Grundfläche von Vorteil. In gleicher Art und Weise wird hierdurch auch das Reinigen der Grundfläche von Verunreinigungen nach dem bestimmungsgemäßen Gebrauch erleichtert.

Weiterhin ist vorgesehen, dass der zumindest eine Kanal im Wesentlichen geradlinig verläuft. Von Vorteil ist weiterhin, wenn der zumindest eine Kanal ein divergierendes, d.h. verbreiterndes oder ein verjüngendes Profil aufweist. Durch die sich über den Verlauf des Kanals ändernde Kanalbreite kann der Transportmechanismus in vielfältiger Weise optimiert werden. Eine sich ändernde Kanalbreite wird hierbei durch einen sich kontinuierlich ändernden Abstand unmittelbar benachbarter, den Kanal bildender Reinigungselemente realisiert.

Weiterhin ist vorgesehen, dass der zumindest eine Kanal gekrümmt verläuft. Hierfür kommt insbesondere ein kreisbogenförmiger Verlauf oder ein spiralartiger Verlauf infrage. Auf diese Art und Weise kann der Transport von Ablagerungen entlang der Grundfläche auch schräg zur Putz- oder Reinigungsrichtung des Mundhygienegeräts verlaufen.

Der Verlauf und die Ausgestaltung des durch Zwischenräume zwischen einzelnen Reinigungselementen gebildeten Kanals sind insbesondere von der Grundgeometrie der an der Grundfläche angeordneten Reinigungselemente abhängig. Die für den Materialtransport maßgeblichen Faktoren sind insbesondere der Abstand und die Flächendichte der jeweiligen Reinigungselemente sowie die bevorzugte Bewegungsrichtung des Mundhygienegeräts beim Reinigungsvorgang und die konkrete Ausgestaltung der zu reinigenden Oberfläche, wie etwa die Zungenoberfläche.

Nach einer weiteren Ausführungsform ist vorgesehen, dass zumindest zwei Reinigungsstäbchen mit ihrem längsseitigen Endabschnitt an gegenüberliegenden Seiten des Kanals zu liegen kommen. So kann der Kanal überwiegend, wenn nicht sogar vollständig von solchen Zwischenräumen gebildet werden, die in Längsrichtung zwischen Reinigungsstäbchen liegen.

Demzufolge sind zumindest einige der an der Grundfläche angeordneten Reinigungsstäbchen derart zueinander angeordnet und ausgerichtet, dass sich im Wesentlichen senkrecht zu ihrer jeweiligen Längserstreckung der für den Abtransport von Ablagerungen vorgesehene Kanal bildet. Die unmittelbar mit ihrem längsseitigen Endabschnitt gegenüberliegend an den Kanal angrenzenden Reinigungsstäbchen sind vorzugsweise parallel zueinander ausgerichtet.

Alternativ oder ergänzend hierzu können die seitlich des Kanals angeordneten Reinigungsstäbchen auch zueinander weitgehend spiegelsymmetrisch zum Kanalverlauf ausgerichtet und angeordnet sein.

Nach einer weiteren Ausführungsform nimmt die Längserstreckung der Reinigungsstäbchen im Verlauf des Kanals zu. Hierdurch kann ein Materialtransport schräg zur Längsrichtung der Reinigungsstäbchen in vorteilhafter Weise verbessert und optimiert werden. Die Anordnung und Ausgestaltung der einzelnen Reinigungselemente, insbesondere der Reinigungsstäbchen können hierbei an eine von Haus aus inhomogene Verteilung und Anhaftung von Ablagerungen und Schmutzpartikeln an der Grundfläche des Mundhygienegeräts angepasst werden. So können z.B. insbesondere an den Bereichen der Grundfläche, an denen sich die von den Reinigungsnoppen gelösten Ablagerungen bevorzugt anlagern, die Reinigungsstäbchen länger und die Kanalbreite zunehmend größer ausgebildet werden.

Je nach Ausgestaltung der zu reinigenden Gewebeoberfläche des Mundinnenraums können jedoch auch alternative und umgekehrte Ausgestaltungen und Anordnungen einzelner Reinigungselemente von Vorteil sein.

Nach einer weiteren vorteilhaften Ausführungsform nimmt der Krümmungsradius eines gekrümmten Kanals im Verlauf des Kanals ab, wodurch insbesondere ein Kanal mit einem spiralartigen Verlauf entsteht.

Nach einer Weiterbildung ist insbesondere vorgesehen, dass Reinigungsnoppen insbesondere in einem äußeren Randbereich und das Reinigungsstäbchen in einem Zentralbereich der Grundfläche angeordnet sind. Eine derartige Anordnung ist insbesondere für die Reinigung der über die Zunge inhomogen verteilten unterschiedlich ausgestalteten Papillen von Vorteil. Weiterhin sorgt eine derartige Anordnung dafür, dass die von den Reinigungsnoppen losgelösten Schmutzpartikel und Ablagerungen sich bevorzugt an den zentral gelegenen Reinigungsstäbchen beim Reinigungsvorgang anlagern. Daneben kann durch eine angewinkelte Schabführung nur entlang der Noppen, deren Wirkung verstärkt werden.

Mithin kann hierdurch eine Transportrichtung für die Schmutzpartikel und Ablagerungen zur Mitte der Grundfläche hin unterstützt bzw. bestimmt werden. Dies ist insbesondere für die Reinigung von Zungen-Randbereichen von Vorteil, da die außen liegenden Bereiche der Grundfläche des Mundhygienegeräts im Wesentlichen nur zum Loslösen, nicht aber zum Abtransport von Verunreinigungen und Ablagerungen vorgesehen sind. Hierdurch kann effektiv vermieden werden, dass die in einem Zungen-Randbereich losgelösten Partikel seitlich von der zu reinigenden Oberfläche herunter geschoben werden. Die zentrale Anordnung der Reinigungsstäbchen trägt somit zu einer verbesserten Aufnahme von gelösten Schmutzpartikeln und Ablagerungen bei.

Nach einer weiteren Ausführungsform ist vorgesehen, dass Reinigungsstäbchen nach Art einer Fischgrät-Anordnung abwechselnd mit bevorzugt etwa gleichen Winkeln aber in umgekehrter Richtung bezogen auf die Querrichtung des Kopfteils zueinander ausgerichtet sind. Alternativ verändern sich die zwischen den Schenkeln eingeschlossenen Winkel. Ferner sind in Längsrichtung benachbart zu liegende kommende Reinigungsstäbchen entlang der Längsachse des Kopfteils voneinander beabstandet und in Querrichtung des Kopfteils bereichsweise überlappend angeordnet.

Die schräge und unterschiedliche Anordnung einzelner Reinigungsstäbchen bezüglich der Längsachse des Kopfteils ist für eine multidirektionale Reinigungswirkung bei einer unidirektionalen Bewegungsrichtung des Mundhygienegeräts von Vorteil. Durch die in Querrichtung des Kopfteils bereichsweise überlappende Anordnung der Reinigungsstäbchen kann insbesondere ein zur Mitte der Grundfläche hingerichteter Transport von Ablagerungen erfolgen.

Nach einer weiteren Ausführungsform sind die Reinigungsstäbchen vom Rand des Kopfteils her zum Zentrum und zum freien Ende des Kopfteils hin ausgerichtet, wobei unter dem freien Ende des Kopfteils dasjenige Ende zu verstehen ist, welches dem Griffteil gegenüberliegend bzw. vom Griffteil abgewandt angeordnet ist. In Anbetracht einer unidirektionalen Bewegungsrichtung des Mundhygienegeräts bei der Zungenreinigung, bei welcher das Mundhygienegerät vorzugsweise beginnend von einem hinteren, tief in der Mundhöhle liegenden Zungenbereich zum vorderen Zungenbereich bewegt wird, erfolgt ein Materialtransport in Richtung zum freien Ende des Kopfteils des Mundhygienegeräts.

Nach einer weiteren vorteilhaften Ausführungsform der Erfindung sind die Reinigungselemente an ihrem freien, der Grundfläche abgewandten Endabschnitt abgerundet und im Wesentlichen parallel zur Grundfläche abgeflacht ausgebildet. Die abgeflacht ausgebildete Stirnfläche der Reinigungselemente dient hierbei als Kontaktfläche, die beim Reinigungsvorgang im Wesentlichen vollständig an der zu reinigenden Oberfläche der Zunge anliegt.

Weiterhin ist vorgesehen, dass das abgerundete freie Ende nach Art eines Kreisbogens zwischen einer seitlichen Wangenfläche und der stirnseitigen abgeflachten Kontaktfläche ausgebildet ist. Diese abgerundeten Kanten der Reinigungsstäbchen und Reinigungsnoppen ermöglichen ein sanftes Entlanggleiten des Mundhygienegeräts über die zu reinigende Oberfläche, wodurch abrupte und ruckartige Bewegungen infolge von Verkantungen von scharfkantigen Ecken des Reinigungsgeräts mit Innenmundgewebe vermieden werden können. Das Mundhygienegerät weist demzufolge kein aggressives Schaberverhalten auf, sondern ermöglicht durch die funktionsgerechte Gestaltung der unterschiedlichen Reinigungselemente, Reinigungsstäbchen und Reinigungsnoppen ein schonendes und zugleich höchst effektives Reinigen der Zungenoberfläche.

Für das abgerundete freie Ende von Reinigungselementen ist insbesondere ein Kreisbogen in einem Winkelbereich von 60° bis 120° vorgesehen.

Weiterhin ist vorgesehen, dass der Radius des Kreisbogens 10 % bis 50 %, insbesondere zwischen 25% - 40%, des Durchmessers oder der Breite des jeweiligen Reinigungselements beträgt.

Weiterhin ist vorgesehen, dass die Höhe des abgerundeten Endbereichs von Reinigungselementen zwischen 15 % und 40 % der Gesamthöhe des jeweiligen Reinigungselements beträgt. Der Radius des Kreisbogens der Abrundung liegt zwischen 02, mm - 0,5 mm. Derartige runde und abgeflachte Ausgestaltungen sämtlicher Reinigungselemente sind insbesondere zur Verringerung des Verletzungsrisikos bei der Reinigung, aber auch für ein sanftes und schonendes Entlanggleiten des Mundhygienegeräts über die zu reinigende Oberfläche ausgebildet.

Nach einem weiteren Aspekt der Erfindung weisen zumindest einige der Reinigungsnoppen in der Ebene parallel zur Grundfläche eine kreisförmige oder elliptische Querschnittsfläche auf. Der Durchmesser von zylindrisch ausgebildeten Reinigungsnoppen bzw. die Elliptizität der Querschnittsfläche der Reinigungsnoppen ist insbesondere an die Ausrichtung und Größe der zu reinigenden Papillen angepasst.

Weiterhin ist vorgesehen, dass zumindest einige der Reinigungselemente in der Ebene senkrecht zur Grundfläche eine viereckige Querschnittsfläche aufweisen. Für die Reinigungsnoppen ist hierbei insbesondere eine quadratische Grundfläche und für die Reinigungsstäbchen eine rechteckige Querschnittsfläche vorgesehen.

Nach einer weiteren Ausführungsform weisen Reinigungsnoppen eine in der Ebene parallel zur Grundfläche rautenartige Querschnittsfläche auf, wobei das Längenverhältnis der Rautendiagonalen zwischen 0,2 und 5, insbesondere zwischen 1 und 3,5 beträgt.

Weiterhin kann vorgesehen sein, dass zumindest eine der Seitenflächen oder Wangenflächen von Reinigungsnoppen und/oder Reinigungsstäbchen konvex und/oder konkav gewölbt ist.

Des Weiteren können seitliche Begrenzungen oder Seitenkanten der Reinigungselemente über nahezu die gesamte Höhe der Reinigungselemente abgerundet ausgebildet sein. Dies ist insbesondere bei rechteckigen oder dreieckigen Grundgeometrien von Reinigungselementen von Vorteil, da somit nicht nur das freie Ende der Reinigungselemente, sondern sämtliche auch im Wesentlichen parallel zur Flächennormalen der Grundfläche verlaufenden Kanten und Ecken der Reinigungselemente abgerundet ausgebildet sind. Dies trägt zu einer weiteren Verringerung eines etwaigen Verletzungsrisikos bei.

Nach einer weiteren Ausführungsform weisen zumindest einige der Reinigungselemente Seitenflächen auf, die zur Flächennormalen der Grundfläche unterschiedlich geneigt sind. Hierfür sind insbesondere derartige Ausgestaltungen von Reinigungselementen vorgesehen, die ein in der Ebene senkrecht zur Grundfläche im Wesentlichen dreieckig ausgebildetes Querschnittsprofil aufweisen. In dieser Anordnung ist ein der Grundfläche abgewandter und spitz zulaufender Endbereich des Reinigungselements vorzugsweise ebenfalls abgerundet ausgebildet.

Weiterhin ist vorgesehen, dass zumindest einige der Reinigungselemente, insbesondere Reinigungsnoppen kegelstumpfartig ausgebildet sind. In besonders vorteilhafterweise sind hierbei die kegelstumpfartig ausgebildeten Reinigungsnoppen als ein entlang seiner Längsrichtung geschnittener und mit dieser Schnittfläche auf der Grundfläche liegender Kegelstumpf ausgebildet. Hierdurch entsteht ein Reinigungselement mit einer halbkreisförmigen Seitenfläche und einer spitz zulaufenden Halbkegelfläche. Der Übergang von Halbkegel-Mantelfläche und der halbkreisförmigen Seitenfläche ist hierbei ebenfalls abgerundet.

Weiterhin ist vorgesehen, dass Reinigungsnoppen eine kugelartige bzw. halbkugelartige Grundgeometrie mit einem im Wesentlichen schräg zur Grundfläche verlaufenden und an die Grundfläche direkt angrenzenden konkav gewölbten Seitenfläche aufweisen. Auch hier ist der Übergang der konkav gewölbten Seitenfläche zur Kugeloberfläche vorzugsweise abgerundet ausgebildet. Die konkave Wölbung kann hierbei unterschiedlich, so zum Beispiel als eine Wölbung mit der Flächennormalen der Grundfläche als Mittelpunkt, aber auch beispielsweise senkrecht dazu ausgebildet sein.

Nach einer weiteren bevorzugten Ausführungsform der Erfindung ist die Breite oder der Durchmesser der Reinigungselemente geringer oder gleich deren Höhe. Ein Höhen- zu Breiten-Verhältnis größer als 1 ermöglicht insbesondere die Verwendung eines relativ weichen Kunststoffmaterials für die Reinigungselemente, welches für eine schonende und wenig strapazierende Reinigung der Zungenoberfläche von Vorteil ist.

So ist insbesondere vorgesehen, dass die Breite der Reinigungselemente mindestens 0,5 mm, vorzugsweise zumindest 0,6 mm beträgt.

Nach einer weiteren Ausführungsform ist vorgesehen, dass die Höhe der Reinigungselemente mindestens 0,6 mm, vorzugsweise zumindest 0,7 mm beträgt.

Nach einem weiteren vorteilhaften Aspekt der Erfindung beträgt die Grundfläche der Reinigungsnoppen zumindest 0,2 mm², vorzugsweise jedoch nicht weniger als 0,25 mm². Die Grundfläche der Reinigungsnoppen bezeichnet diejenige Querschnittsfläche der Reinigungsnoppen, die in der Grundfläche des Kopfteils liegt.

Weiterhin ist vorgesehen, dass die maximale Querschnittsfläche der im Schnitt kreisförmigen Reinigungsnoppen 0,8 mm² beträgt. Alle Reinigungselemente/noppen weisen eine max. Querschnittfläche von 4 mm², insbesondere 3 mm² auf

Nach einer weiteren Ausführungsform beträgt in demjenigen Flächensegment der Grundfläche, in welchem die Reinigungsnoppen angeordnet sind, die Summe der Grundflächen (begrenzt durch die äußersten Reinigungsnoppen) sämtlicher Reinigungsnoppen bis zu 75 %, insbesondere 10-50% der Fläche dieses Flächensegments. Folglich sind die einzelnen Reinigungsnoppen relativ dicht und in einem dementsprechend geringen Abstand von beispielsweise 0,2 mm bis 0,3 mm voneinander beabstandet angeordnet.

Damit ist unabhängig von der Putzrichtung eine Reinigungswirkung gegeben.

Nach einer weiteren Ausführungsform sind zumindest einige der Reinigungselemente spiegelsymmetrisch zur Längsachse des Griffteils ausgerichtet und/oder angeordnet.

Weiterhin kann vorgesehen werden, dass die Reinigungselemente, hierunter insbesondere die Reinigungsstäbchen und/oder die Reinigungsnoppen punktsymmetrisch zum Mittelpunkt der Grundfläche bzw. zum Flächenmittelpunkt des Kopfteils angeordnet sind. Die spiegelsymmetrische bzw. punktsymmetrische Anordnung von Reinigungselementen betrifft nicht nur deren Position, sondern vor allem auch die Orientierung der Reinigungselemente bezüglich der Längsachse des Griff- oder Kopfteils.

Nach einer weiteren Ausführungsform ist vorgesehen, dass die Reinigungsstäbchen zumindest bereichsweise eine in einem Winkelbereich von -30°, insbesondere 10° bis +60° (Hinterschnitt) insbesondere 45° zur Flächennormalen der Grundfläche geneigte Wangen- oder Seitenfläche aufweisen. Demzufolge können die Reinigungsstäbchen nicht nur als rechteckige und quaderförmige Stäbchen, sondern auch als Strukturen mit einem parallelogrammartig oder trapezoidartig ausgebildeten Querschnittsprofil ausgebildet sein.

Die Seitenflächen können hierbei sowohl zum freien Ende der Stäbchen verjüngend als auch verbreiternd ausgebildet sein. Bei letzterer Ausgestaltung bilden die Wangen- oder Seitenflächen der Reinigungsstäbchen zumindest eine Hinterschneidung, die zur Aufnahme von Ablagerungen und Schmutzpartikeln der Zungenoberfläche von Vorteil sein kann. Hierbei wird eine Verbreiterung bevorzugt. Eine Hinterschneidung wird nur in Verbindung mit einem Weichkunststoffmaterial gewünscht.

Nach einem weiteren Aspekt der Erfindung beträgt in demjenigen Flächensegment der Grundfläche, in welchem die Reinigungsstäbchen angeordnet sind, die Summe der Querschnittsflächen sämtlicher Reinigungsstäbchen parallel zur Grundfläche zwischen 5 Prozent und 50 Prozent der Fläche dieses Flächensegments. Im Vergleich zur Anordnung der Reinigungsnoppen sind die Reinigungsstäbchen vorzugsweise in einem größeren Abstand voneinander an der Grundfläche des Mundhygienegeräts angeordnet. Einerseits sind somit die zur Aufnahme von Verunreinigungen vorgesehenen Zwischenräume zwischen den Reinigungsstäbchen größer. Andererseits ermöglicht dies eine bessere Verteilung und einen besseren Transport von Schmutzpartikeln über der Grundfläche. In gleicher Weise wird hierdurch auch die Reinigung der Grundfläche nach erfolgter Reinigungsprozedur im Mundinnenraum verbessert und erleichtert.

Nach einer weiteren Ausführungsform ist vorgesehen, dass die minimale Breite des zumindest einen Kanals 0,5, insbesondere 0,8 mm, und die maximale mittlere Breite des zumindest einen Kanals 2 mm, insbesondere 1,5 mm beträgt.

Weiterhin ist vorgesehen, dass die Längserstreckung des zumindest einen Kanals mindestens 5 mm beträgt. Auf diese Art und Weise kann mittels des zumindest einen Kanals ein Materialtransport im Wesentlichen senkrecht zur Längserstreckung der Reinigungsstäbchen über eine Strecke von zumindest 5 mm erfolgen.

Nach einem weiteren Aspekt der Erfindung ist in dem vom Griffteil abgewandten Abschnitt der Grundfläche zumindest ein geknickt verlaufendes Reinigungsstäbchen mit zwei im Wesentlichen senkrecht zueinander ausgerichteten Schenkeln angeordnet. Hierbei ist jeder der Schenkel nach Art eines geradlinig verlaufenden Reinigungsstäbchens mit im Wesentlichen rechteckiger Grundgeometrie ausgebildet. Die beiden rechtwinklig zueinander angeordneten Schenkel sind vorzugsweise auf der Längsachse des Kopfteils einstückig miteinander verbunden. Hierbei erstrecken sich die beiden Schenkel jeweils von der mittig entlang des Kopfteils verlaufenden Längsachse schräg nach außen und in Richtung zum Griffteil.

Nach einem weiteren vorteilhaften Aspekt der Erfindung sind als Reinigungselemente in der Ebene parallel zur Grundfläche gekrümmt verlaufende Reinigungsbögen vorgesehen. Derartige Reinigungsbögen, die vorzugsweise ebenfalls an ihrem freien Endabschnitt abgerundet ausgebildet sind, haben die Funktion von Zungenschaberelementen, die insbesondere im Bereich des freien Endes des Kopfteils zu liegen kommen. Diese mitunter wandartig ausgebildeten Reinigungsbögen verlaufen mit ihren Seiten- oder Wangenflächen vornehmlich geneigt zur Flächennormale der Grundfläche.

Es ist hierbei insbesondere vorgesehen, dass die zum Griffteil hin angeordnete Wangenfläche von Reinigungsbögen in einem Winkel von 85° bis 100° zur Grundfläche verläuft, so dass der Reinigungsbogen ggf. eine Hinterschneidung bildet, in welcher Ablagerungen und Schmutzpartikel während des Reinigungsvorgangs angesammelt werden können.

Es ist weiterhin vorgesehen, dass zumindest zwei entlang der Längsachse des Griffs voneinander beabstandete Reinigungsbögen und/oder zumindest zwei geknickt verlaufende Reinigungsstäbchen an der Grundfläche des Mundhygienegeräts angeordnet sind.

Hierbei ist insbesondere vorgesehen, dass die Höhe der bogenartigen bzw. geknickt verlaufenden Reinigungselemente unterschiedlich ist und insbesondere zum Zentrum des Kopfteils hin zunimmt. Derartig unterschiedlich hoch ausgebildete und in ihrer Funktion schabende Reinigungselemente tragen dem Aspekt Rechnung, dass die zu reinigende Zungenoberfläche nicht als gerade, sondern bereichsweise gekrümmte Oberfläche ausgebildet ist. Durch die unterschiedlich hoch ausgebildeten Reinigungselemente ist ferner ein an die Zungenstruktur angepasstes und besonders schonendes Reinigen möglich.

Nach einem weiteren Aspekt der Erfindung bestehen sämtliche Reinigungselemente aus einem thermoplastischen Elastomer, welches eine Shore-Härte von 30 bis 70 A, insbesondere von 40 bis 60 A aufweist. Ein derartig flexibles Kunststoffmaterial ermöglicht ein komfortables und für den Anwender angenehmes Reinigen der Zunge oder etwa auch des Zahnfleischs. Hinsichtlich des elastischen Kunststoffmaterials sei an dieser Stelle auf die geometrischen Abmessungen und die Dimensionierung der einzelnen Reinigungselemente verwiesen. Die Kombination eines Höhen- zu Breiten-Verhältnisses größer als 1 mit Abmessungen im Sub-Millimeterbereich und einer Shore-Härte von 40 bis 60 A beugt Verletzungen vor und stellt insbesondere durch die Implementierung von Reinigungselementen mit unterschiedlicher Geometrie und Größe zugleich eine effektive Reinigungswirkung für weiches Körpergewebe im Mundinnenraum zur Verfügung.

Nach einer weiteren vorteilhaften Ausführungsform der Erfindung ist vorgesehen, dass .die aus einem thermoplastischen Elastomer bestehenden Reinigungselemente einstückig mit der am Kopfteil vorgesehenen Grundfläche verbunden sind. Hierbei ist weiterhin von Vorteil, dass die aus einem thermoplastischem Elastomer gebildete Grundfläche nahtlos und einstückig zumindest in die Rückseite des Griffteils übergeht.

Nach einem weiteren vorteilhaften Aspekt weist das Kopfteil zumindest zwei unterschiedliche Kunststoffmaterialien bevorzugt unterschiedlicher Elastizität auf. Neben der vorzugsweise aus einem thermoplastischen Elastomer gebildeten Grundfläche besteht der Kern des Kopfteils vorzugsweise aus einem härteren Kunststoff, wie beispielsweise Polypropylen. Das aus Polypropylen gebildete Kernelement des Mundhygienegeräts sorgt somit aufgrund seiner relativ unflexiblen und steifen Beschaffenheit für eine unmittelbare und direkte Kontrolle des Mundhygienegeräts während des Reinigungsvorgangs. So wird ein vom Benutzer ausgeübter Druck im Wesentlichen unverfälscht und direkt auf die zu reinigende Fläche im Mundinneren übertragen.

Nach einer weiteren Ausführungsform ist vorgesehen, dass das die Grundfläche bildende Kunststoffmaterial das Kopfteil an seiner Unterseite und an den Seitenflächen nahezu vollständig umschließt. Da das Mundhygienegerät vorzugsweise mit seiner Unterseite des Kopfteils zur Reinigung der Zunge ausgebildet ist, gelangt eine im Wesentlichen geschlossene und mit Reinigungselementen versehene Fläche mit der Zungenoberfläche in Berührung.

Diese Ausführungsform ist insoweit vorteilhaft, weil die Rückseite und die Seitenflächen des Zahnbürstenkopfes in der Mundhöhle komfortabler bewegbar sind.

Weiterhin ist vorgesehen, dass das härtere Kunststoffmaterial im Bereich des Kopfteils zumindest einen Vorsprung aufweist, welcher in die vom weicheren Kunststoff gebildete Grundfläche flächenbündig eingebettet ist. Der Vorsprung erstreckt sich hierbei im Wesentlichen senkrecht zur Grundfläche und ragt sozusagen in diese hinein.

Weiterhin ist vorgesehen, dass der in die Grundfläche hineinragende Vorsprung eine hellere Farbgebung und/oder einen geringeren optischen Absorptionsgrad als die Grundfläche selbst aufweist. Auf diese Art und Weise kann somit auch während des Reinigungsvorgangs der Grad der Verschmutzung der Grundfläche des Kopfteils ohne große Probleme und unmittelbar vom Anwender visuell beobachtet und erfasst werden.

Nach einem weiteren vorteilhaften Aspekt der Erfindung ist vorgesehen, dass im Zentrum der Grundfläche zumindest ein Flächenabschnitt vorgesehen ist, der zur Aufnahme eines Reinigungsmediums ausgebildet ist. Dieser für das Reinigungsmedium vorgesehene Flächenabschnitt ist im Wesentlichen eben und ohne vorspringende Reinigungselemente ausgebildet.

Zudem kann vorgesehen werden, dass der zur Aufnahme des Reinigungsmediums vorgesehene Flächenabschnitt eine Vertiefung für das Reinigungsmedium aufweist oder gänzlich als ein gegenüber der Grundfläche vertiefter Abschnitt ausgebildet ist. Die Anordnung eines zur Aufnahme eines Reinigungsmediums vorgesehenen Flächenabschnitts in der Mitte der Grundfläche ist für eine gleichmäßige Verteilung des Reinigungsmediums an der zu reinigenden Zungenoberfläche besonderes geeignet.

Nach einem weiteren unabhängigen Aspekt betrifft die Erfindung ein Mundhygienegerät, bei welchem die der Grundfläche gegenüberliegende Vorderseite des Kopfteils als ein mit Zahnbürstenborsten bestückter Zahnbürstenkopf ausgebildet ist. Insofern kann das insbesondere für die Zungenreinigung vorgesehene erfindungsgemäße Mundhygienegerät mit einer Zahnbürste kombiniert oder in eine Zahnbürste integriert werden.

### Ausführungsbeispiele

Weitere Ziele, Merkmale sowie vorteilhafte Anwendungsmöglichkeiten der vorliegenden Erfindung ergeben sich aus der nachfolgenden Beschreibung von Ausführungsbeispielen anhand der Zeichnungen. Dabei bilden sämtliche beschriebenen und/oder bildlich dargestellten Merkmale für sich oder in beliebiger sinnvoller Kombination den Gegenstand der vorliegenden Erfindung, auch unabhängig von den Patentansprüchen oder deren Rückbeziehung.

Es zeigen:
- Figur 1: die schematische Darstellung einer Ausführungsform des Mundhygienegeräts in Draufsicht,
- Figur 2: eine perspektivische Darstellung des Mundhygienegeräts gemäß Figur 1,
- Figur 3: eine weitere perspektivische Darstellung des Mundhygienegeräts gemäß Figur 1,
- Figur 4: eine perspektivische Darstellung eines Reinigungsstäbchens gemäß der Ausführungsform der Figuren 1 bis 3,
- Figur 5: das Reinigungsstäbchen gemäß Figur 4 im Querschnitt,
- Figur 6: das Reinigungsstäbchen gemäß Figur 4 in einer weiteren perspektivischen Darstellung,
- Figur 7: das Reinigungsstäbchen gemäß Figur 4 längs geschnitten,
- Figur 8: eine perspektivische Darstellung einer Reinigungsnoppe gemäß dem Ausführungsbeispiel nach Figur 1,
- Figur 9: die Reinigungsnoppe gemäß Figur 8 in einer Seitenansicht,
- Figur 10: die Reinigungsnoppe gemäß Figur 8 im Querschnitt,
- Figur 11: eine perspektivische Darstellung eines weiteren Ausführungsbeispiels mit trapezartig ausgebildeten Reinigungselementen,
- Figur 12: eine vergrößerte perspektivische Darstellung des Reinigungselements gemäß Figur 11,
- Figur 13: die perspektivische Darstellung einer halbkegelförmigen Reinigungsnoppe,
- Figur 14: eine perspektivische Darstellung einer halbkugelförmigen Reinigungsnoppe,
- Figur 15: eine Seitenansicht der halbkugelförmigen Reinigungsnoppe gemäß Figur 14,
- Figur 16: die perspektivische Darstellung einer im Querschnitt herzförmigen Reinigungsnoppe,
- Figur 17: eine weitere perspektivische Darstellung der Reinigungsnoppe gemäß Figur 16 schräg von oben,
- Figur 18: die perspektivische Darstellung eines elliptisch ausgebildeten Reinigungselements,
- Figur 19: eine weitere perspektivische Darstellung des Reinigungselements gemäß Figur 18 mit elliptischer Querschnittsfläche,
- Figur 20: die perspektivische Darstellung eines rautenförmig ausgestalteten Reinigungselements,
- Figur 21: eine weitere perspektivische Darstellung des Reinigungselements gemäß Figur 20,
- Figur 22: eine perspektivische Darstellung eines an den Kanten abgerundeten Reinigungselements quadratischen Querschnitts,
- Figur 23: ein weiteres Ausführungsbeispiel eines Zungenreinigers mit insgesamt drei verschiedenartig ausgebildeten Reinigungselementen,
- Figur 24: eine perspektivische Darstellung des Reinigungselements gemäß Figur 23 von schräg oben,
- Figur 25: eine weitere perspektivische Darstellung des Zungenreinigers gemäß Figur 23 schräg von der Seite,
- Figur 26: eine schematische und perspektivische Darstellung dreier geknickt und winklig verlaufender Reinigungsstäbchen,
- Figur 27: ein weiteres Ausführungsbeispiel eines Zungenreinigers mit Zungenschaberelementen,
- Figur 28: eine perspektivische Ansicht des Ausführungsbeispiels gemäß Figur 27,
- Figur 29: eine weitere perspektivische Ansicht des Ausführungsbeispiels gemäß Figur 27 schräg von der Seite,
- Figur 30: ein weiteres Ausführungsbeispiel einer zungenreinigenden Struktur,
- Figur 31: die perspektivische Darstellung des Zungenreinigers gemäß Figur 30,
- Figur 32: eine weitere Konfiguration von Reinigungsstäbchen und Reinigungsnoppen an einer Grundfläche in Aufsicht,
- Figur 33: eine perspektivische Darstellung der Struktur gemäß Figur 32,
- Figur 34: eine weitere perspektivische Darstellung der Struktur gemäß Figur 32 schräg seitlich,
- Figur 35: eine weitere spiralartig angeordnete Reinigungsstruktur mit Reinigungsstäbchen und Reinigungsnoppen in Aufsicht,
- Figur 36: eine Zungenreinigerkonfiguration mit in Art eines Fischgrätmusters angeordneten Reinigungsstäbchens,
- Figur 37: eine perspektivische Darstellung des Zungenreinigers gemäß Figur 36,
- Figur 38: eine weitere perspektivische Darstellung des Zungenreinigers gemäß Figur 36 schräg von der Seite,
- Figur 39: die Darstellung einer weiteren Reinigungsstäbchenkonfiguration in Aufsicht
- Figur 40: die Konfiguration gemäß Figur 39 in einer Ansicht schräg von oben und
- Figur 41: eine Seitenansicht einer Zahnbürste,
- Figur 42: eine Draufsicht der Zahnbürste nach Figur 41 und
- Figur 43: eine Rückansicht der Zahnbürste nach Figur 41.

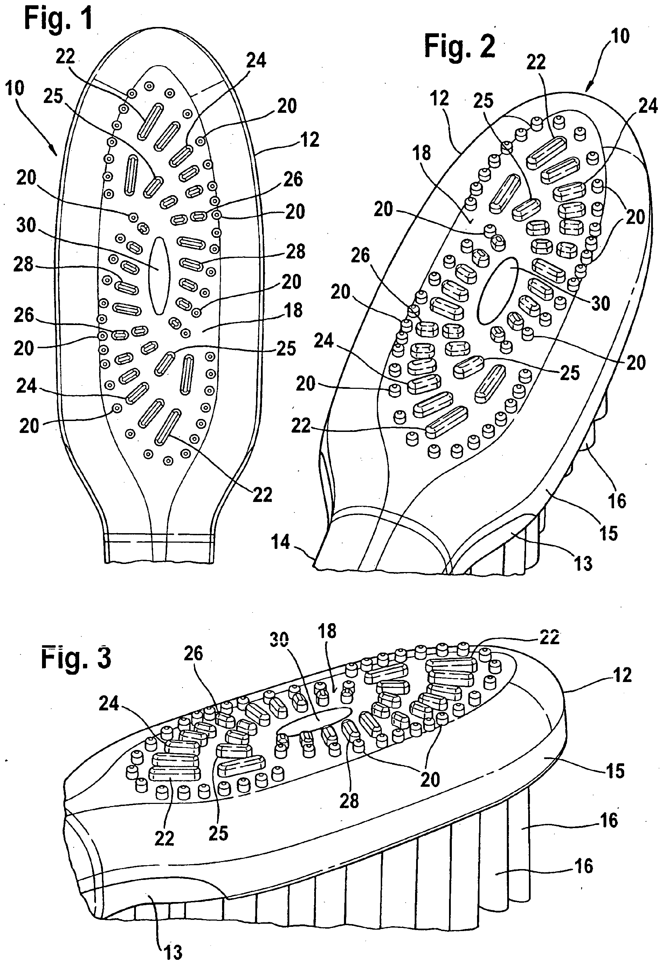

In den Figuren 1 bis 3 ist ein erstes Ausführungsbeispiel des Mundhygienegeräts 10 in Form eines Zungenreinigers mit einem Kopfteil 12 und einem an das Kopfteil angrenzenden Griffteil 14 dargestellt. An dem Kopfteil 12 ist eine im Wesentlichen ebene Grundfläche 18 vorgesehen, an welcher zumindest zwei unterschiedliche Typen bzw. Arten von vorspringenden Reinigungselementen 20, 22, 24, 25, 26, 28 angeordnet sind.

Als Reinigungselemente kommen bei diesem Ausführungsbeispiel zum einen zylindrisch ausgebildete Reinigungsnoppen 20 und mit unterschiedlicher Längserstreckung ausgebildete längliche und geradlinig verlaufende Reinigungsstäbchen 22, 24, 25, 26, 28 in Betracht.

Abhängig von ihrer jeweiligen geometrischen Ausgestaltung sind die einzelnen Reinigungselemente 22, 24, 25, 26, derart an der Grundfläche 18 angeordnet, dass sich zumindest ein quer zur Längsrichtung von Reinigungsstäbchen 22, 24, 25, 26, verlaufender und aus Zwischenräumen zwischen unmittelbar benachbart zu liegen kommenden Reinigungselementen 22, 24, 25, 26, ein Kanal bildet. Dieser ist insbesondere für eine homogene Verteilung und einen Transport von Ablagerungen und Schmutzpartikeln vorgesehen, die beim Reinigungsvorgang vom Zungenreiniger 10 aufgenommen werden.

Das Ausführungsbeispiel gemäß der Figuren 1 und 3 weist mehrere quer zur Längsrichtung von Reinigungsstäbchen 22, 24, 25, 26, verlaufende Kanäle auf. So beginnt beispielsweise ein erster, in Figur 1 nach rechts unten gekrümmt verlaufender Kanal in Höhe des Reinigungsstäbchens 26. Dieser verläuft dann zwischen den Reinigungsstäbchen 24 und 25 und endet schließlich in Höhe des in Griffnähe angeordneten Reinigungsstäbchens 22.

In ähnlicher Art und Weise verläuft ein weiterer Reinigungskanal rechts oberhalb des zentrisch angeordneten Vorsprungs 30 ebenfalls in Höhe des Stäbchens 26 und unter einer Verbreiterung seiner Kanalbreite gekrümmt nach oben links.

Die einzelnen seitlich an den Kanal angrenzenden Reinigungsstäbchen weisen mit zunehmendem Verlauf entlang des Kanals eine zunehmende Längserstreckung auf. Zudem ändert sich die Ausrichtung benachbarter, an den Kanal angrenzender Reinigungsstäbchen 22, 24, 26, 28 sukzessive um einen bestimmten veränderlichen Winkel, so dass letztlich das erste Reinigungsstäbchen 26 und das letzte Reinigungsstäbchen 22 des Reinigungskanals um 30° bis 80° zueinander gedreht angeordnet sind. Größere Winkelbereiche, in etwa größer 90° oder gar 180° sind ohne weiteres denkbar.

Durch die unterschiedliche Ausrichtung der einzelnen Reinigungsstäbchen 22, 24, 25, 26, 28 kann selbst bei einer unidirektionalen Reinigungsbewegung, wie zum Beispiel bei einer geradlinigen Vor- und Zurück-Bewegung auch ein Reinigungseffekt quer zur Bewegungsrichtung des Mundhygienegeräts 10 erzielt werden.

Die in den Figuren 1 bis 3 gezeigte Anordnung von Reinigungselementen 20, 22, 24, 25, 26, 28 ist zudem im wesentlichen punktsymmetrisch zum Mittelpunkt des zentrisch angeordneten Vorsprungs 30. Dieser in die Grundfläche 18 integrierte Vorsprung 30 ist einstückig mit dem Kernmaterial 13 des Kopfteils 12 verbunden. Die gesamte in den Figuren 1 bis 3 dargestellte Rückseite des Kopfteils 12 ist hingegen mit einer Schicht aus einem weichen Kunststoffmaterial, wie etwa einem thermoplastischen Elastomer überzogen, die insbesondere auch den seitlichen Rand 15 des Kopfteils 12 umschließt.

Die die Grundfläche 18 bildende Elastomerschicht geht zudem nahtlos in die Rückseite des Griffteils 14 über. Im Bereich des Halses 13 gibt die das Kopfteil 12 umschließende Elastomerschicht 18 den härteren Kernbereich des Kopf- und des Griffteils 14 frei, welcher insbesondere aus einem harten Kunststoffmaterial, wie etwa Polypropylen gefertigt ist. Der einstückig mit dem Kernmaterial 13 verbundene Vorsprung 30 durchstößt die Grundfläche 18 und schließt flächenbündig mit dieser ab. Der Vorsprung 30 ist sozusagen in die Grundfläche 18 integriert und ist von einzelnen Reinigungselementen 28 seitlich umgeben.

Das seitliche Übergreifen des Kopfteils 12 mit der Elastomerschicht sowie die Einbettung des vom Kernmaterial vorspringend ausgebildeten Vorsprungs 30 in die Grundfläche 18 ist für das Zusammenfügen und das Zusammenhalten der unterschiedlichen Kunststoffe, insbesondere im Hinblick auf die Bildung einer formschlüssigen Verbindung zwischen Grundfläche 18 und Vorsprung von Vorteil.

Die in den Figuren 1 bis 3 nicht explizit dargestellte Vorderseite des Kopfteils 12 kann insbesondere zur Anbringung einer Vielzahl von Zahnbürstenborsten 16 ausgebildet sein, so dass dem Mundhygienegerät eine Doppelfunktion als Zahnbürste und als Zungenreiniger zukommen kann.

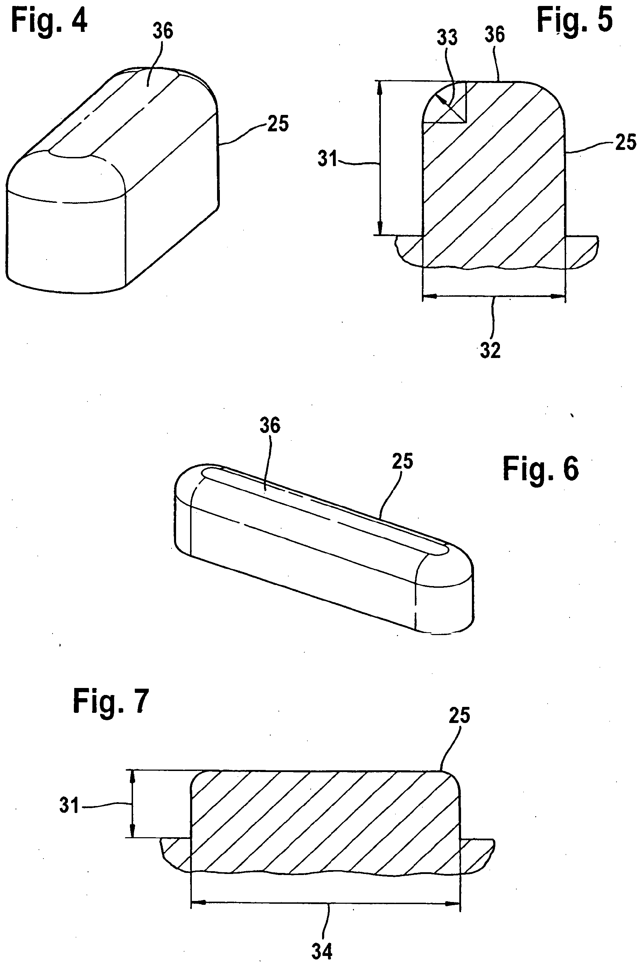

In den Figuren 4 bis 6 ist ein Reinigungsstäbchen 25 beispielhaft in verschiedenen Ansichten dargestellt. Das Reinigungsstäbchen 25 weist an seinem freien, der Grundfläche 18 abgewandtem Endabschnitt eine im Wesentlichen parallel zur Grundfläche verlaufende abgeflachte Kontaktfläche 36 sowie abgerundete Kantenbereiche auf. Des Weiteren ist das Verhältnis von Höhe 31 zu Breite 32 sämtlicher Reinigungsstäbchen 22, 24, 25, 26, 28 größer oder gleich 1.

So ist insbesondere vorgesehen, dass die Höhe der Reinigungsstäbchen mindestens 0,6 mm, vorzugsweise aber zumindest 0,7 mm beträgt, während die Breite der Reinigungsstäbchen 22, 24, 25, 26, 28 mindestens 0,5 mm, vorzugsweise zumindest 0,6 mm beträgt. Der einen Übergang zwischen Seitenfläche und stirnseitiger Kontaktfläche 36 bildende abgerundete Bereich dient einem leichten und ruckfreien Gleiten des Mundhygienegeräts entlang der zu reinigenden Fläche.

Die nahezu in jeder Richtung abgerundeten Reinigungsstäbchen dienen vornehmlich einem Transport von bereits von der Zungenoberfläche losgelösten Ablagerungen und Schmutzpartikeln. Auf diese Art und Weise kann einerseits ein Verletzungsrisiko des Zungengewebes minimiert und der Abtransport von Ablagerungen aus der Mundhöhle optimiert werden.

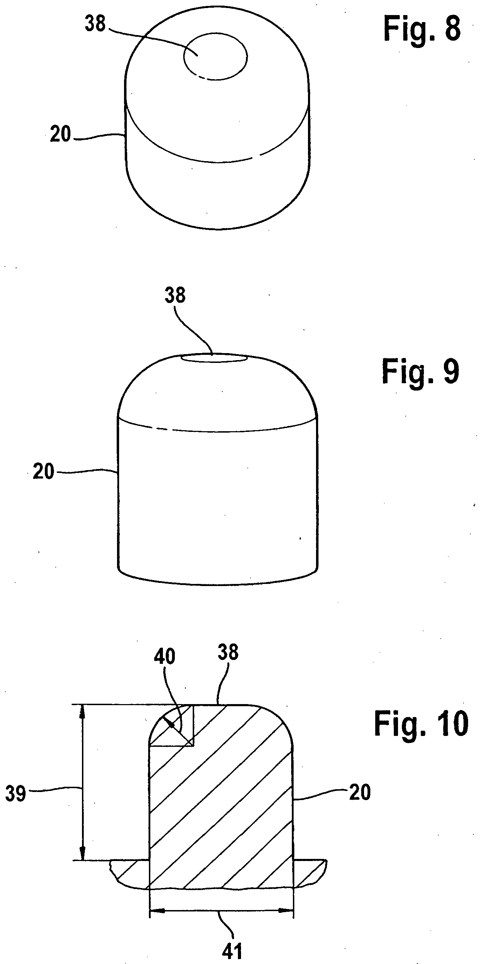

In den Figuren 8 bis 10 ist eine zylindrisch ausgebildete Reinigungsnoppe exemplarisch dargestellt. Auch die Reinigungsnoppen verlaufen an ihrem der Grundfläche 18 abgewandten Endabschnitt abgerundet und haben eine an die Abrundung angrenzende abgeflachte Kontaktfläche 38.

Der Krümmungsradius 40 der Reinigungsnoppen 20 als auch der Krümmungsradius 33 der Reinigungsstäbchen 22, 24, 25, 26, 28 liegt in einem Bereich zwischen 10 % und 50 % insbesondere zwischen 25% und 40% der Breite 32 eines Reinigungsstäbchens 22, 24, 25, 26, 28 bzw. 10 % und 50 % insbesondere zwischen 25% und 40% des Durchmessers 41 einer zylindrisch ausgebildeten Reinigungsnoppe 20.

Das Verhältnis von Höhe 39 zu Durchmesser 41 der Reinigungsnoppen 20 entspricht im Wesentlichen dem Höhe- und Breite-Verhältnis der Reinigungsstäbchen 22, 24, 25, 26, 28.

Im Gegensatz zur Funktion der Reinigungsstäbchen 22, 24, 25, 26, 28 sind die Reinigungsnoppen 20 zum Eintauchen in Oberflächenvertiefungen oder Zwischenräume der Zunge ausgebildet, um dort Schmutzpartikel oder sonstige Ablagerungen loszulösen. Die unterschiedlich ausgestalteten Reinigungselemente, nämlich Reinigungsnoppen 20 und Reinigungsstäbchen 22, 24, 25, 26, 28 sind somit für unterschiedliche Funktionen ausgelegt und weisen dementsprechend unterschiedliche Geometrien auf.

Einzig die Höhe von Reinigungsstäbchen 22, 24, 25, 26, 28 und Reinigungsnoppen 20 ist in etwa gleich. Zumindest ragen die Noppen 20 nicht über die Reinigungsstäbchen 22, 24, 25, 26, 28 hinaus.

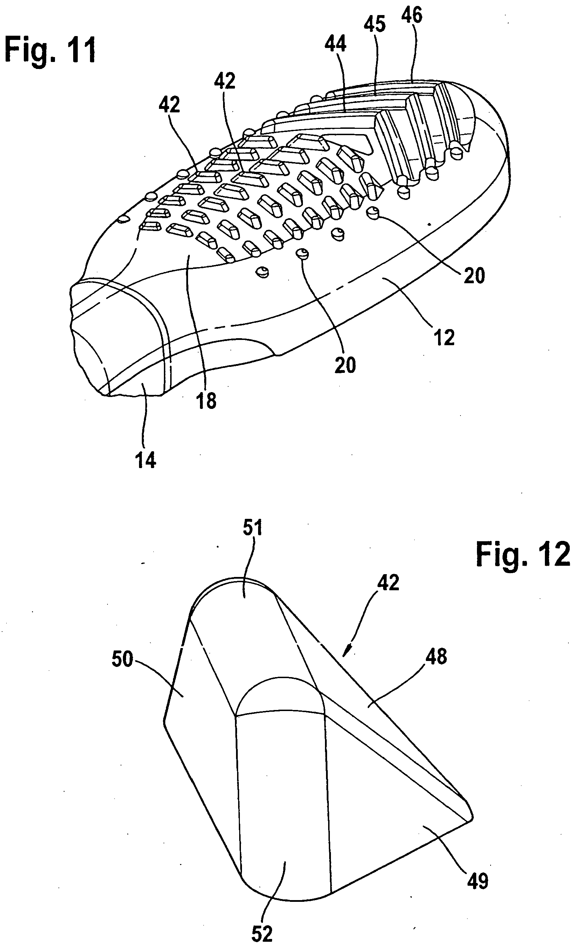

Figur 11 zeigt ein weiteres Ausführungsbeispiel mit fächerförmig angeordneten Reinigungselementen 42, die eine in etwa dreieckige Querschnittsfläche parallel zur Grundfläche 18 aufweisen. Die im Wesentlichen spitz zulaufenden und an ihrem freien Ende abgerundet ausgebildeten Reinigungselemente 42, welche in Figur 12 in einer detaillierten perspektivischen Darstellung gezeigt sind, weisen insbesondere unterschiedlich geneigte Wangen- oder Seitenflächen 50, 48 auf.

Die insbesondere spitzwinklig aufeinander zulaufenden Seiten- oder Wangenflächen 50, 48 sind über Abrundungen 51, 52 miteinander verbunden.

In dem Ausführungsbeispiel gemäß Figur 11 vergrößert sich die Längserstreckung der einzelnen Reinigungselemente 42 mit zunehmendem Abstand vom Griffteil 14. Des Weiteren sind in dem Bereich des freien, dem Griffteil 14 gegenüberliegend angeordneten Endabschnitts des Kopfteils 12 drei geknickt und abgewinkelt verlaufende Reinigungsstäbchen 44, 45, 46 angeordnet. Sie sind ferner mit jeweils zwei im Wesentlichen senkrecht zueinander ausgerichteten Schenkeln versehenen. Diese geknickt verlaufenden Reinigungsstäbchen dienen einem Aufsammeln von Rückständen, Ablagerungen und Schmutzpartikeln, die zuvor während des Reinigungsvorgangs von den einzelnen Reinigungselementen 42 von der Zungenoberfläche gelöst wurden.

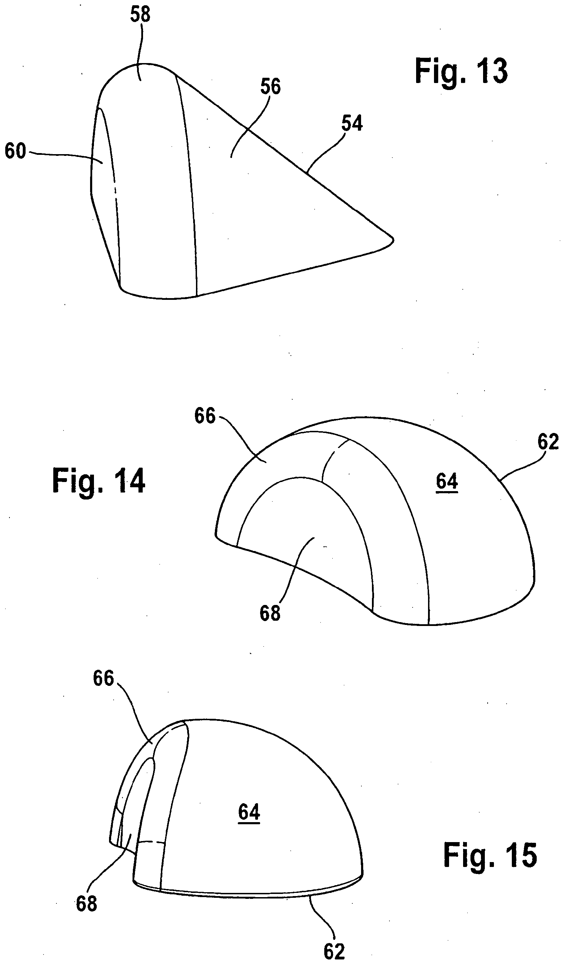

Figur 13 zeigt ein weiteres Ausführungsbeispiel einer Reinigungsnoppe 54, die nach Art eines halbierten Kegelstumpfs ausgebildet ist. Eine halbkreisförmige Wangen- oder Stirnfläche 60 ist im Wesentlichen senkrecht zur Grundfläche 18 angeordnet, während die sich über eine Abrundung 58 daran anschließende Kegelmantelfläche 56 mit auslaufender Höhe in die Grundfläche 18 übergeht. Diese nach Art eines liegenden und halbierten Kegelstumpfes ausgebildete Reinigungsnoppe 54 erweist sich dann als vorteilhaft, wenn ein Reinigungseffekt lediglich in einer Bewegungsrichtung des Mundhygienegeräts 10 auf die Zunge ausgeübt werden soll.

Ist beispielsweise die Wangen- oder Stirnfläche 60 bei einer Reinigungsbewegung der Mantelfläche 56 vorauseilend, so wird losgelöstes Material zumindest von der Stirnfläche 60 weiter geschoben oder gelöst. Bei einer umgekehrten Bewegungsrichtung der Reinigungsnoppe 54 ist hingegen aufgrund des stetigen Anstiegs der Reinigungsnoppe hin zur Abrundung 58 kaum ein Reinigungseffekt zu erwarten. Durch die relativ zur Längsachse verschieden winklige Anordnung der Reinigungselemente erlaubt insbesondere bei einer halbierten, liegenden Kegelstumpf-Form, Pyramidenstumpf-Form des Elementes verschiedene wirksame Reinigungsflächen.

In den Figuren 14 und 15 ist ein weiteres Ausführungsbeispiel einer kugelförmigen Reinigungsnoppe 64 dargestellt. Diese nach Art einer Halbkugel ausgebildete Reinigungsnoppe 64 liegt nahe ihres maximalen Querschnitts an der Grundfläche 18 auf, so dass nur die sich nach oben mit abnehmendem Querschnitt erstreckende Halbkugel aus der Grundfläche 18 hervorragt.

Weiterhin ist die halbkugelförmige Reinigungsnoppe 64 mit einer nach innen gewölbten Wangenfläche 68 versehen, die sich vom Fuß der Reinigungsnoppe 64 bis nahezu an ihre höchste Erhebung oberhalb der Grundfläche 18 erstreckt. Insofern kann die kugelförmige Reinigungsnoppe 64 auch annähernd als Viertel kugel beschrieben werden, deren ebene, das heißt, nicht kugelförmige Seite eine konkave Wölbung aufweist. Der Übergang der konkaven Wölbung 68 zur konvex ausgebildeten Kugeloberfläche ist wiederum als Abrundung 66 ausgebildet, so dass auch hier ein etwaiges Verletzungsrisiko minimiert werden kann.

Ähnlich wie das in Figur 13 gezeigte Ausführungsbeispiel stellt auch die halbkugelartig ausgebildete Reinigungsnoppe eine im Wesentlichen unidirektionale Reinigungswirkung zur Verfügung.

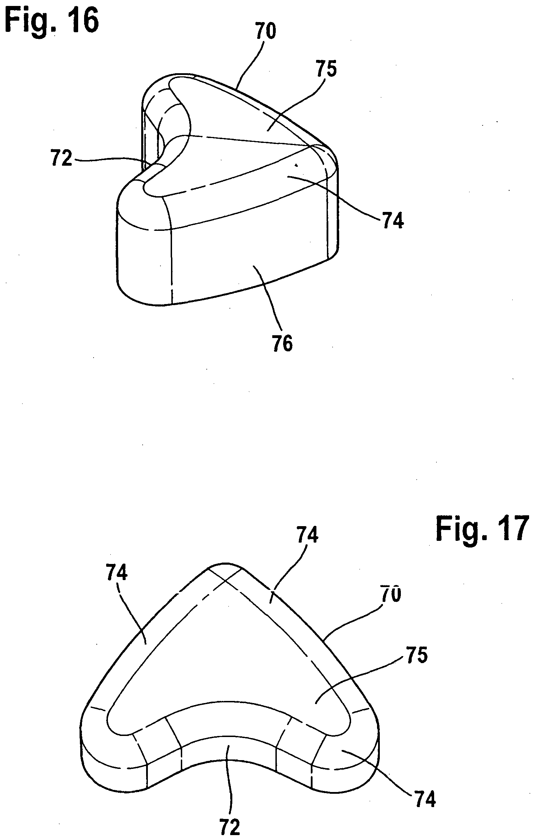

Ein weiteres Ausführungsbeispiel einer Reinigungsnoppe 70 zeigen die Figuren 16 und 17 in unterschiedlichen perspektivischen Darstellungen. Die Grundfläche der Reinigungsnoppe 70 ähnelt einer Herz-Form, die in erster Näherung als ein Dreieck mit abgerundeten Ecken, zwei nach außen gewölbten und einer nach innen gewölbten Seitenwandung beschrieben werden kann.

Die konkav nach innen gewölbte Wangen- oder Seitenfläche 72 als auch die beiden konvex, nach außen gewölbten Wangen- oder Seitenflächen 76 der Reinigungsnoppe 70 verlaufen im Wesentlichen senkrecht zur Ebene der Grundfläche 18. Der Übergang der Seitenflächen 72, 76 zur im Wesentlichen parallel zur Grundfläche 18 verlaufenden Kontaktfläche 75 ist wiederum nicht als Kante, sondern in Form einer Abrundung 74 realisiert.

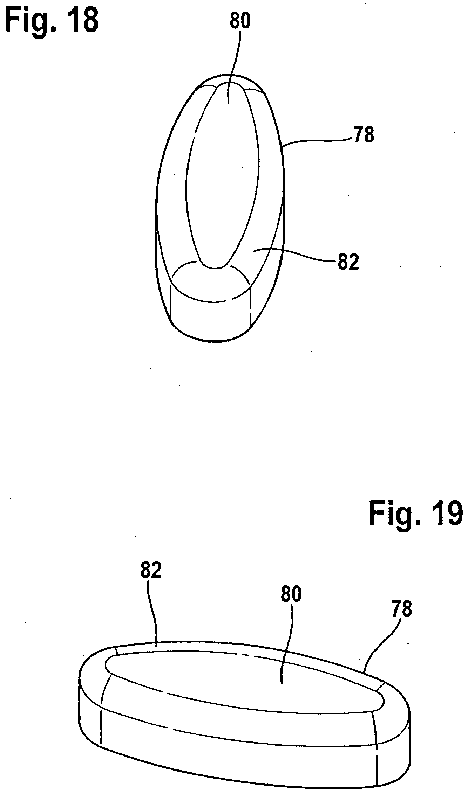

Die Figuren 18 und 19 zeigen ein weiteres Reinigungselement 78, welches sowohl als elliptische Reinigungsnoppe als auch als ein entlang der Längsachse symmetrisch gewölbtes Reinigungsstäbchen im Sinne dieser Erfindung beschreiben werden kann. Die Seitenflächen verlaufen auch hier, ähnlich wie bei der Dreiecksform gemäß der Figuren 16 und 17 im Wesentlichen senkrecht zur Grundfläche 18. Der obere, an die Kontaktfläche 80 heranreichende Bereich ist abermals als eine den oberen Randbereich umlaufende Abrundung zwischen Kontaktfläche und Seitenflächen ausgebildet.

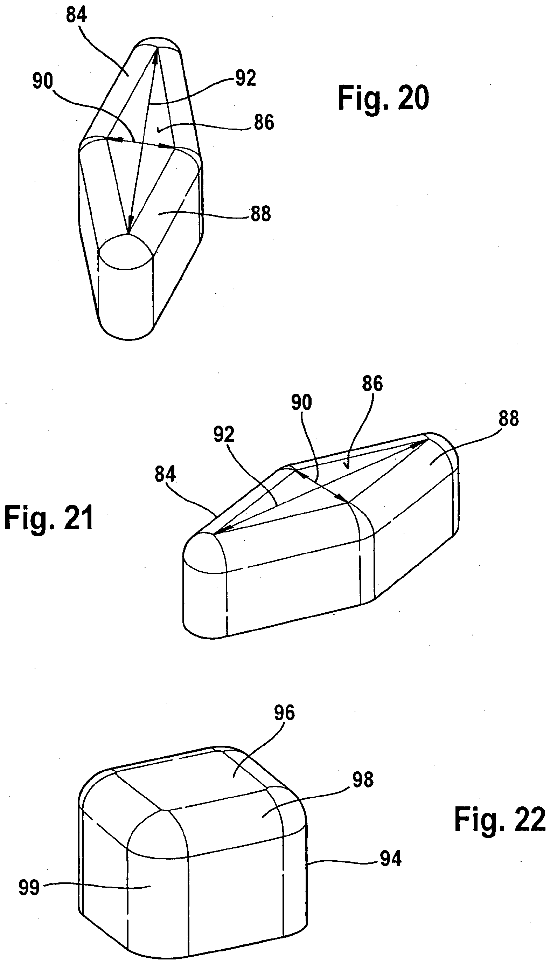

Die Figuren 20 und 21 zeigen ferner eine Reinigungsnoppe 84 in unterschiedlichen perspektivischen Darstellungen, die eine rautenartig ausgebildete Grundfläche aufweist. Derartige Reinigungsnoppen 84 können in unterschiedlichen Ausgestaltungen und Geometrien verwirklicht werden. Das Längenverhältnis der beiden Diagonalen 90, 92 kann zwischen 0,2 und 5 betragen.

Figur 22 zeigt weiterhin eine perspektivische Darstellung einer würfelartig ausgebildeten Reinigungsnoppe 94, die an ihrem der Grundfläche 18 abgewandten freien Ende eine abgeflachte Kontaktfläche 96 aufweist. Zudem sind sämtliche Ecken und aneinander angrenzenden Seiten als Abrundungen 98, 99 ausgebildet.

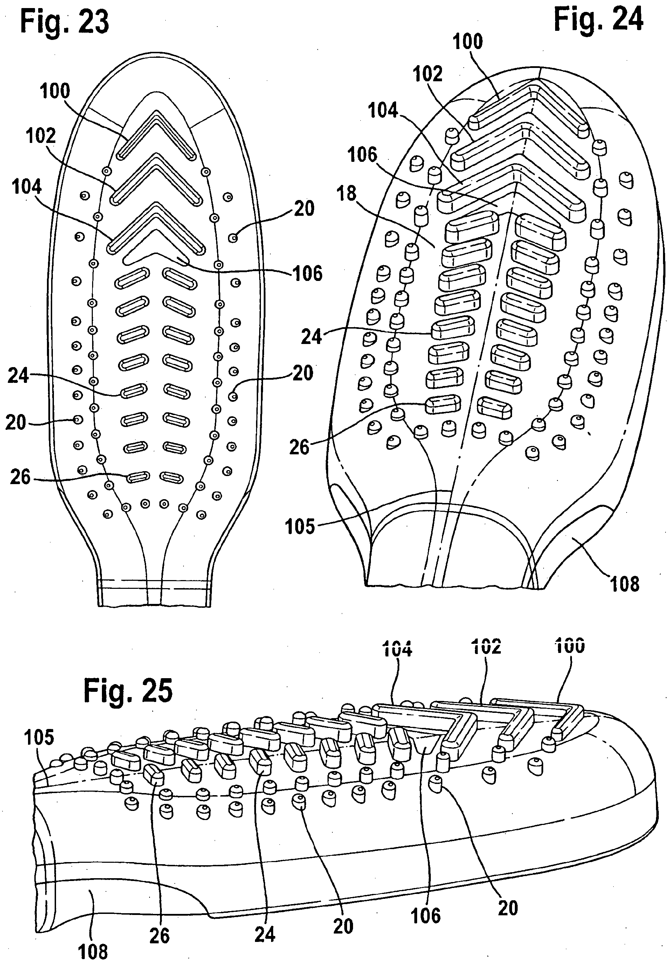

Die Figuren 23 bis 25 zeigen ein weiteres Ausführungsbeispiel des Mundhygienegeräts, bei welchem drei unterschiedliche Typen von Reinigungselementen, nämlich Reinigungsnoppen 20, Reinigungsstäbchen 24, 26 und drei geknickt verlaufende Reinigungsstäbchen 100, 102, 104 mit unterschiedlicher Höhe angeordnet sind. Die gesamte Anordnung von Reinigungselementen 20, 24, 26, 100, 102, 104 ist spiegelsymmetrisch zu Längsachse 105 des Griffteils bzw. des Kopfteils ausgebildet.

Die Reinigungsnoppen 20 sind entlang der Längsseiten in zwei Reihen versetzt zueinander angeordnet, während die schräg zur Querachse des Kopfteils verlaufenden Reinigungsstäbchen 24, 26 paarweise auf gleicher Höhe angeordnet nach innen und zum freien Kopfende hin verlaufend zu liegen kommen. Die Längserstreckung der einzelnen Reinigungsstäbchen 26, 24 nimmt zum freien Ende des Kopfteils 12 hin zu.

Im oberen Endabschnitt des Kopfteils 12 sind die drei geknickt verlaufenden Reinigungsstäbchen 100, 102, 104 entlang der Längsachse 105 des Kopfteils 12 versetzt zueinander angeordnet. Jedes der geknickt verlaufenden Reinigungsstäbchen 100, 102, 104 weist zwei zueinander um etwa 90° gedreht angeordnete und miteinander in Höhe der Längsachse 105 des Kopfteils 12 einstückig verbundene stäbchenartig ausgebildete Seitenschenkel auf. Jedes der einzelnen geknickt verlaufenden Reinigungsstäbchen 100, 102, 104 hat ein etwa konstantes Höhenprofil.

Die drei geknickten Reinigungsstäbchen 100, 102, 104 sind unterschiedlich hoch ausgebildet. Das Reinigungsstäbchen 100 hat eine geringere Höhe als das unmittelbar benachbart zu liegen kommende Reinigungsstäbchen 102, welches wiederum eine geringere Höhe als das am weiten innen liegendste geknickte Reinigungsstäbchen 104 aufweist. Zwischen den Schenkeln des geknickt verlaufenden Reinigungsstäbchens 104 und den geradlinig verlaufenden Reinigungsstäbchen 24, 26 kommt der einstückig mit dem Kernmaterial 108 des Kopfteils verbundene Vorsprung 106 flächenbündig mit der Grundfläche 18 zu liegen.

Abgesehen von den vorteilhaften Befestigungsmöglichkeiten, für die unterschiedlichen Kunststoffmaterialien des Kopfteils bietet der in der Grundfläche 18 eingebettete Vorsprung 106 bei Verwendung eines z.B. weißen oder gelben Kunststoffs die Möglichkeit, den Verschmutzungsgrad des Zungenreinigers während der Reinigungsanwendung in einfacher und zuverlässiger Weise visuell zu überprüfen.

Darüber hinaus bildet derjenige Abschnitt, in welchem der Vorsprung 106 zu liegen kommt eine zentralen Bereich innerhalb der Grundfläche 18, der keine Reinigungselemente aufweist, sondern lediglich von solchen umgeben ist. Von daher eignet sich der um den Vorsprung herum angeordnete Flächenabschnitt auch als Aufnahme für ein Reinigungsmedium.

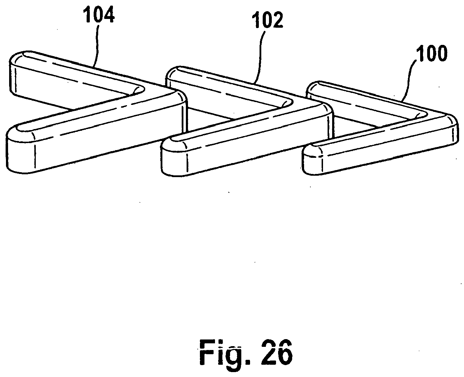

Figur 26 zeigt noch einmal in einer vergrößerten Darstellung und in einer seitlichen perspektivischen Ansicht die unterschiedlich hoch ausgebildeten geknickt bzw. winklig verlaufenden Reinigungsstäbchen 100, 102, 104 gemäß der Ausführungsform nach den Figuren 23 bis 25.

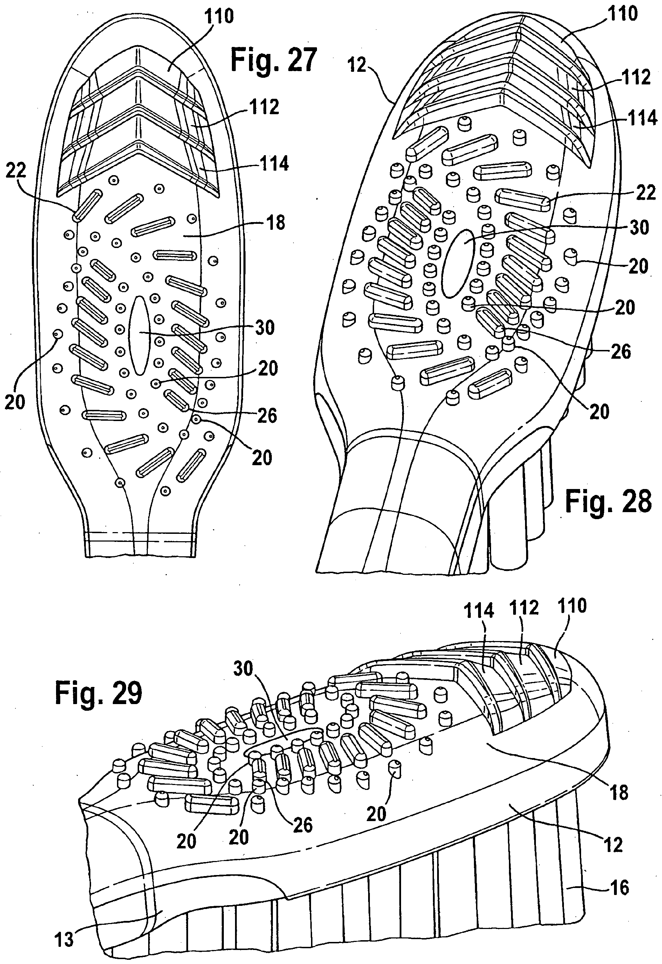

In den Figuren 27 und 28 sind eine Aufsicht und eine perspektivische Darstellung einer weiteren Ausführungsform des Zungenreinigers dargestellt. Auch hier finden insgesamt drei verschiedenartig strukturierte Reinigungselemente 20, 22, 26, 110, 112, 114 Verwendung. Jedes Reinigungsstäbchen 22, 26 ist hierbei mit jeweils zwei Reinigungsnoppen 20 gepaart, wobei jeweils eine Reinigungsnoppe 20 in der Verlängerung der Längserstreckung eines jeden Reinigungsstäbchens 22, 26 in einem Abstand zu liegen kommt.

Die einzelnen Reinigungsstäbchen 22, 26 sind nach Art einer Spirale angeordnet, wobei sowohl der Abstand benachbarter Stäbchen 22, 26 als auch deren Orientierung sukzessive mit fortlaufender Spirale anwächst. In ähnlicher Art und Weise vergrößert sich in fast allen Fällen auch der Zwischenraum zwischen den Reinigungsstäbchen 26, 22 und den jeweils in Längsrichtung zugeordneten Reinigungsnoppen 20. Der zum Abtransport von sich an den Reinigungsstäbchen 22, 26 anlagernden Schmutzpartikeln vorgesehene Kanal verläuft jeweils zwischen dem längsseitigen Ende der Reinigungsstäbchen 22, 26 und den dazu in Längsrichtung beabstandet angeordneten Reinigungsnoppen 20.

Die gesamte Anordnung von Reinigungsnoppen 20 und Reinigungsstäbchen 22, 26 ist im Wesentlichen punktsymmetrisch zum Mittelpunkt des in der Grundfläche 18 integrierten Vorsprungs 30 angeordnet. Während sich die links oberhalb des Vorsprungs 30 beginnende Spirale von links oben nach rechts unten krümmt, verläuft die andere aus Reinigungsstäbchen 22, 26 und Reinigungsnoppen 20 gebildete spiralartige Anordnung von rechts unten nach links oben um den in der Ebene elliptisch ausgebildeten Vorsprung 30 herum.

Am freien Ende des Kopfteils 12 sind drei weitere knickartig verlaufende als Schaber oder Sammlerelemente ausgebildete Reinigungsstäbchen 110, 112, 114 angeordnet. Diese weisen ein asymmetrisches Querschnittsprofil mit unterschiedlich steilen Seitenwänden auf. Die den Reinigungsnoppen 20 und geradlinig verlaufenden Reinigungsstäbchen 22, 26 zugewandte Seite der Reinigungsstäbchen 114, 112, 110 verläuft nahezu senkrecht zur Grundfläche 18, während die im freien Ende des Kopfteils 12 hin zugewandte Seiten- oder Wangenfläche in einem relativ flachen Winkel zur Grundfläche ausläuft.

Diese im Wesentlichen über das gesamte Kopfteil 12 quer verlaufenden Reinigungselemente 110, 112, 114 sind insbesondere zum Schaben und Aufsammeln von losgelösten Partikeln ausgebildet.

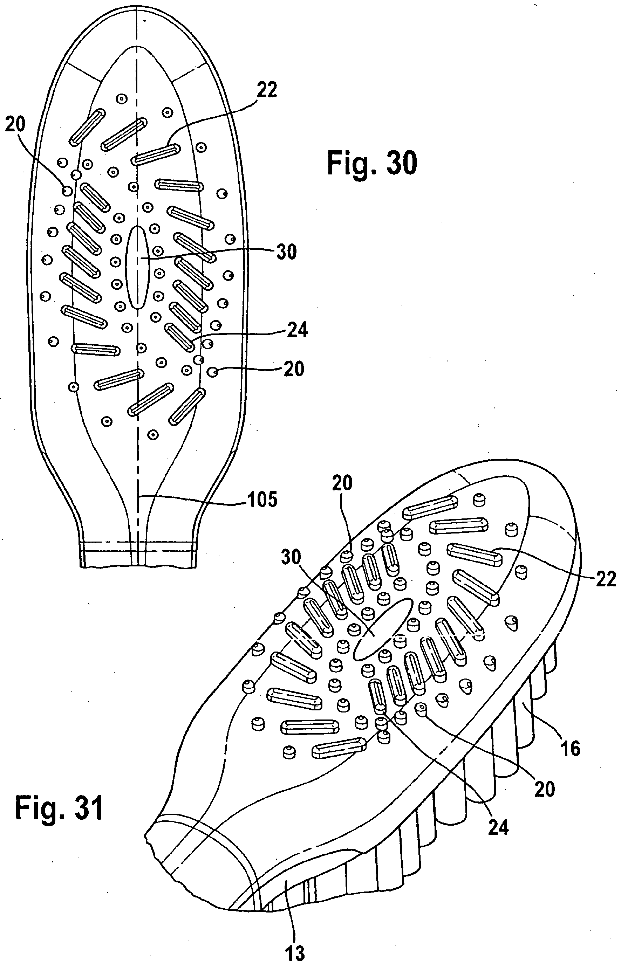

Die Figuren 30 und 31 zeigen ein weiteres Ausführungsbeispiel, welches gegenüber der Ausführungsform gemäß der Figuren 27 bis 29 ohne die am freien Ende des Kopfteils 12 vorgesehenen Reinigungsstäbchen 110, 112, 114 ausgebildet ist. Stattdessen ist die spiralartige Anordnung von paarweise vorgesehenen Reinigungsstäbchen 24, 22 und Reinigungsnoppen 20 in Längsrichtung des Kopfteils 12 in die Länge gestreckt.

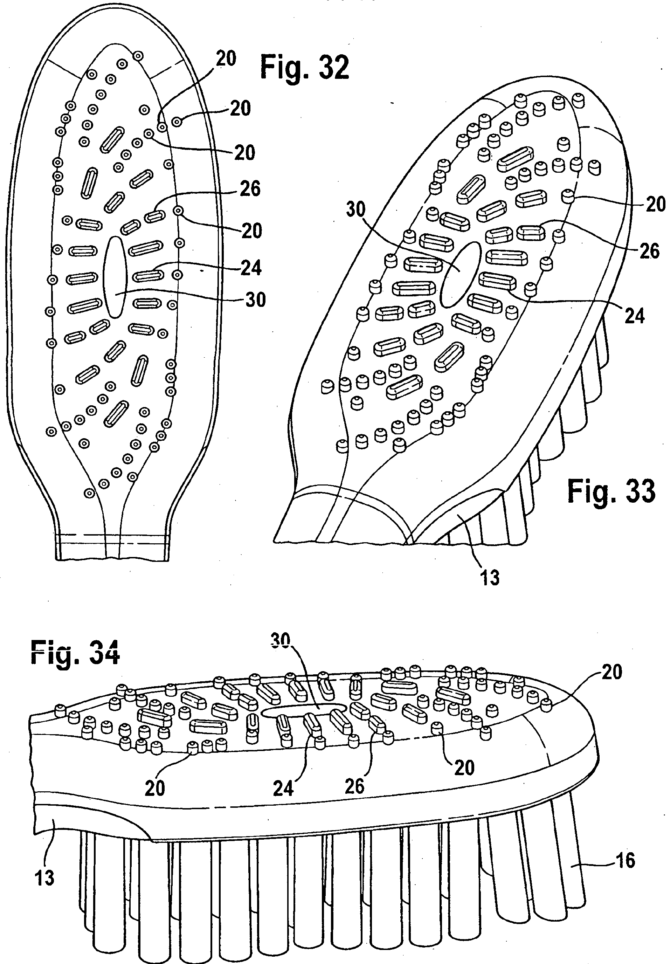

In den Figuren 32 bis 34 ist eine weitere punktsymmetrische Anordnung von Reinigungsstäbchen 24, 26 und Reinigungsnoppen 20 in verschiedenen perspektivischen Ansichten wiedergegeben. Bei dieser Anordnung ist nahezu jedem Reinigungsstäbchen 24, 26 eine außen zu liegen kommende Reinigungsnoppe 20 zugeordnet. Auch hier sind die Reinigungsstäbchen entlang einer Spirale angeordnet, wobei Kanäle entweder zwischen Reinigungsnoppen 20 und Reinigungsstäbchen 24, 26 oder rein zwischen in Längsrichtung voneinander beabstandeten Reinigungsstäbchen 24, 26 vorgesehen sind.

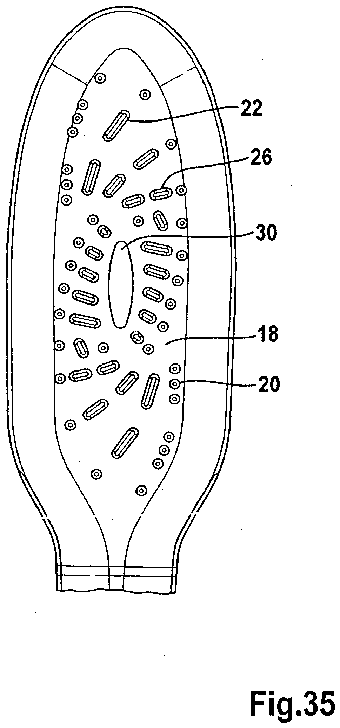

Ein weiteres Beispiel punktsymmetrisch und spiralartig angeordneter Reinigungsstäbchen 26, 22 und Reinigungsnoppen 20 zeigt Figur 35 in einer Draufsicht.

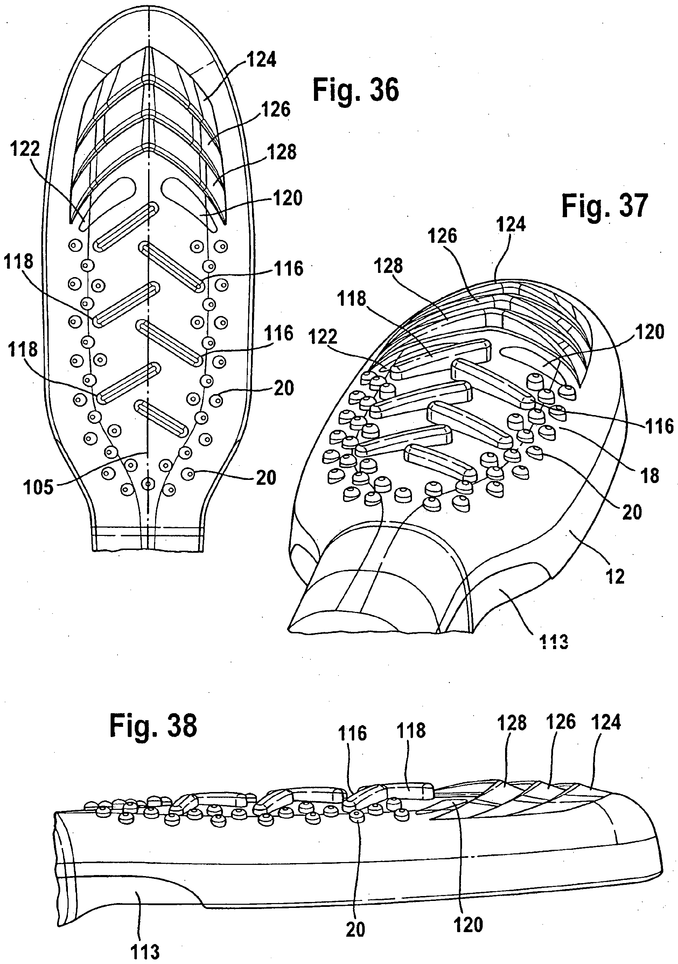

Schließlich ist in den Figuren 36 bis 38 eine weitere Ausführungsform des Kopfteils 12 mit drei unterschiedlich ausgebildeten Reinigungselementen 20, 116, 118 sowie 124, 126, 128 vorgesehen. Die Reinigungsnoppen 20 sind im unteren, dem Griffteil 14 zugewandten Bereich randseitig und versetzt zueinander angeordnet.

Dazwischen liegend sind insgesamt sechs Reinigungsstäbchen 116, 118 vorgesehen, die nach Art eines Fischgrätmuster angeordnet sind, die beginnend vom unteren Kopfteil wechselseitig von außen nach innen und stets zum freien Ende des Kopfteils hin verlaufend angeordnet sind.

Die bezüglich ihrer Ausrichtung spiegelsymmetrisch verlaufenden Reinigungsstäbchen 118, 116 kommen hierbei nicht in gleiche Höhe am Kopfteil 12, sondern jeweils in Längsrichtung versetzt zueinander zu liegen. Die an der rechten Seite des Kopfteils 12 schräg nach innen und oben verlaufenden Reinigungsstäbchen 116 kommen mit ihrem innen liegenden Ende in Längsrichtung bereichsweise überdeckend mit den spiegelsymmetrisch und versetzt an der linken Seite des Kopfteils angeordneten Reinigungsstäbchen 118 zu liegen.

Im Bereich des freien Endes des Kopfteils sind drei lamellen- oder wandartige, leicht gekrümmte bzw. spitzbogenartig ausgebildete Reinigungsstäbchen 128, 126, 124 vorgesehen. Diese sind zur Mitte des Kopfteils hin geneigt, so dass die jeweils zum Zentrum des Kopfteils 12 hin weisende Wangen- bzw. Seitenfläche zur Ansammlung von Ablagerungen und Schmutzpartikeln von Vorteil sein kann.

Des Weiteren ist insbesondere anhand der perspektivischen Darstellung gemäß Figur 37 zu erkennen, dass die Reinigungselemente 20, 116, 118 als auch die lammellenartig ausgebildeten Reinigungselemente 124, 126, 128 in der Mitte des Kopfteils 12 eine größere Höhe als am links- und rechtsseitigen Rand aufweisen. Eine solche Anordnung trägt dem Aspekt Rechnung, dass auch die darunter liegende Grundfläche 18 nicht exakt planar, sondern zu den Seitenrändern des Kopfteils 12 leicht nach unten geneigt verläuft.

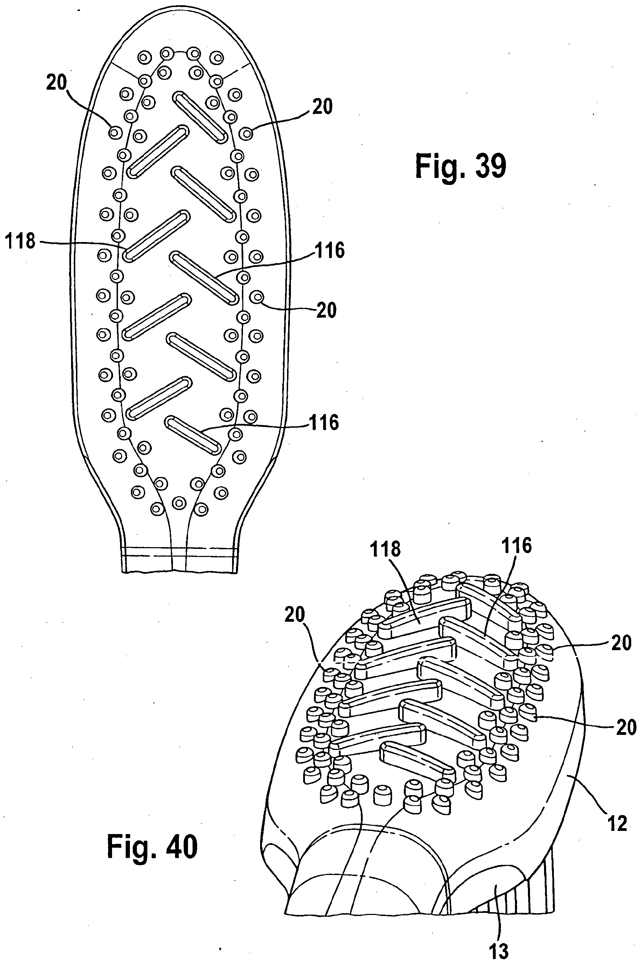

In den Figuren 39 und 40 ist eine weitere an die Ausführungsform gemäß der Figuren 36 bis 38 angelehnte Anordnung von Reinigungselementen 20, 116, 118 gezeigt. Hier bilden die Reinigungsnoppen einen die gesamte Grundfläche umlaufenden Rand, wohingegen die nach Art eines Fischgrätmusters angeordneten Reinigungsstäbchen 116, 118 innerhalb dieses von den Reinigungsnoppen 20 gebildeten Randes zu liegen kommen.

Bei den Ausführungsbeispielen gemäß der Figuren 36 bis 40 verläuft der an die Reinigungsstäbchen 116, 118 angrenzende Kanal S- oder schlangenförmig zwischen den innen liegenden Endabschnitten der einzelnen Reinigungsstäbchen 118, 116.

Vorzugsweise weisen die Ausführungsbeispiele Reinigungselemente auf, deren etwa relativ zur Grundfläche senkrechte Seitenflächen über alle Elemente zusammenaddiert zwischen 50 mm² und 200 mm², insbesondere zwischen 60 mm² und 180 mm² und vor allem zwischen 60 mm² und 120 mm² beträgt, wobei die unteren und oberen Grenzwerte auch vertauscht werden können. Es hat sich gezeigt, dass bei einer Summe aller senkrechten (aktiven) Reinigungsflächen in diesem Bereich optimale Reinigungsergebnisse gegenüber einer sonst ggf. nur massierenden Wirkung oder einer schlechteren Reinigungsleistung erzielbar sind.

Die Figuren 41 bis 43 zeigen ein weiteres Ausführungsbeispiel eines Mundhygienegerätes in Form einer Zahnbürste 200. Dabei ist ein Zungenreiniger 10 wie in Figur 1 auf der Zahnbürstenkopfrückseite 201 vorgesehen (siehe Figur 43). Alternativ kann die Zahnbürste mit einer der anderen zuvor beschriebenen Zungenreiniger oder Elementen davon versehen sein. Die Zahnbürstenkopfvorderseite 204, die in Figur 42 dargestellt ist, weißt ein Borstenfeld 202 mit mehreren Filamentbüscheln 203 und insbesondere je seitlich vom Borstenfeld 202 Zahnfleischmassagestäbe 205, 206 auf. Die Zahnfleischmassagestäbe bzw. Massagefortsätze 205, 206 sind vorzugsweise aus einem TPE (thermoplastischen Elastomer) - Kunststoff ausgebildet und sind somit bevorzugt aus dem gleichen Material wie der Zungenreiniger 10 am PP (Polypropylen) Zahnbürstengrundkörper angespritzt. Der Zahnbürstenkopf ist somit im 2 Komponenten Spritzgiessverfahren hergestellt, wobei ggf. durch eine TPE- Weichkomponenten im Griffbereich eine dritte Komponente oder die gleiche Weichkomponente wie für den Kopf hinzukommt. Hartkomponenten sind in den Figuren 41 bis 43 mit A und Weichkomponenten mit B markiert. Vorteilhaft ist somit der Zungenreiniger 10 und die Zahnfleischmassagestäbe in einem Spritzschritt herstellbar. In der dargestellten Ausführungsform sind an den Bürstenkopfseitenwangen oder/und an der Bürstenkopfrückseite mehrere Ausnehmungen 207 optional vorgesehen. Diese Ausnehmungen 207 sind unterhalb bzw. benachbart zu den langgestreckten Zahnfleischmassagestäben 205, 206 angeordnet. Somit ist die TPE-Kunststoffschrumpfung beim Erkalten nach dem Spritzgießen im Bereich der Ausnehmung durch die geringere Materialstärke an TPE stark verringert, so dass die Massagefortsätze 205, 206 in der vorgesehenen (aufrechten) Position nach dem Spritzgießen bleiben, als dies ohne die Ausnehmungen 207 der Fall ist. Diese Ausbildung mit Ausnehmungen 207 benachbart zu den Massagefortsätzen 205, 206 ist auch unabhängig von einem Zungenreiniger, also auch ohne diesen und bei allen Zahnbürstenarten vorsehbar.

## Patentansprüche

1. Mundhygienegerät mit einem Griffteil (14) und einem Kopfteil (12), an welchem eine im Wesentlichen ebene Grundfläche (18) vorgesehen ist, an der vorspringende Reinigungselemente (20, 22, 24, 25, 26, 28; 42; 54; 62; 70; 78; 84; 94; 100, 102, 104; 110, 112, 114; 116, 118; 124; 126; 128) mit zumindest zwei unterschiedlichen Grundgeometrien angeordnet sind, von denen zumindest einige als längliche Reinigungsstäbchen (22, 24, 25, 26, 28, 116, 118) ausgebildet sind, wobei zumindest ein quer zur Längsrichtung von Reinigungsstäbchen (22, 24, 25, 26, 28, 116, 118) verlaufender und aus Zwischenräumen zwischen unmittelbar benachbart angeordneten Reinigungselementen (20, 22, 24, 25, 26, 28; 42; 54; 62; 70; 78; 84; 94; 100, 102, 104; 110, 112, 114; 116, 118; 124; 126; 128) gebildeter Kanal vorgesehen ist, **dadurch gekennzeichnet, dass** als Reinigungselemente noppenartig ausgebildete Reinigungsnoppen (20) vorgesehen sind und dass in der Verlängerung der Längserstreckung von Reinigungsstäbchen (22, 24, 25, 26, 28; 116, 118) zumindest eine Reinigungsnoppe (20) angeordnet ist.

2. Mundhygienegerät nach einem der vorhergehenden Ansprüche, **dadurch gekennzeichnet, dass** der zumindest eine Kanal im Wesentlichen senkrecht zur Längsrichtung der jeweiligen an den Kanal angrenzenden Reinigungsstäbchen (22, 24, 25, 26, 28; 116, 118) verläuft.

3. Mundhygienegerät nach einem der vorhergehenden Ansprüche, **dadurch gekennzeichnet, dass** zumindest zwei wenigstens bereichsweise schräg zueinander verlaufende Kanäle an der Grundfläche (18) vorgesehen sind.

4. Mundhygienegerät nach einem der vorhergehenden Ansprüche, **dadurch gekennzeichnet, dass** der zumindest eine Kanal geradlinig verläuft.

5. Mundhygienegerät nach einem der vorhergehenden Ansprüche, **dadurch gekennzeichnet, dass** der zumindest eine Kanal divergierend oder verjüngend verläuft.

6. Mundhygienegerät nach einem der vorhergehenden Ansprüche 1 bis 3 oder 5, **dadurch gekennzeichnet, dass** der zumindest eine Kanal gekrümmt verläuft.

7. Mundhygienegerät nach einem der vorhergehenden Ansprüche, **dadurch gekennzeichnet, dass** zumindest zwei Reinigungsstäbchen (22, 24, 25, 26, 28; 116, 118) mit ihrem längsseitigen Endabschnitt an gegenüberliegenden Seiten des zumindest einen Kanals zu liegen kommen.

8. Mundhygienegerät nach einem der vorhergehenden Ansprüche, **dadurch gekennzeichnet, dass** die gegenüberliegend am zumindest einen Kanal zu liegen kommenden Reinigungsstäbchen (22, 24, 25, 26, 28; 116, 118) mit ihrer Längsrichtung im Wesentlichen senkrecht zum angrenzenden Kanal und etwa parallel zueinander ausgerichtet sind.

9. Mundhygienegerät nach einem der vorhergehenden Ansprüche, **dadurch gekennzeichnet, dass** die Längserstreckung der Reinigungsstäbchen (22, 24, 25, 26, 28; 116, 118) im Verlauf des Kanals zunimmt.

10. Mundhygienegerät nach einem der vorhergehenden Ansprüche 1 bis 3 oder 5 bis 9, **dadurch gekennzeichnet, dass** der Krümmungsradius des Kanals im Verlauf des Kanals abnimmt.

11. Mundhygienegerät nach einem der vorhergehenden Ansprüche, **dadurch gekennzeichnet, dass** Reinigungsnoppen (20) in einem äußeren Randbereich und dass Reinigungsstäbchen (22, 24, 25, 26, 28; 116, 118) in einem Zentralbereich der Grundfläche (18) angeordnet sind.

12. Mundhygienegerät nach einem der vorhergehenden Ansprüche, **dadurch gekennzeichnet, dass** Reinigungsstäbchen (116, 118) nach Art eines Fischgrätmusters abwechselnd in unterschiedlichen Richtungen entlang der Längsachse (105) des Kopfteils (12) voneinander beabstandet und in Querrichtung des Kopfteils (12) bereichsweise überlappend angeordnet sind.

13. Mundhygienegerät nach einem der vorhergehenden Ansprüche, **dadurch gekennzeichnet, dass** die Reinigungsstäbchen (22, 24, 25, 26, 28; 116, 118) sich vom Rand des Kopfteils (12) her zum Zentrum und zum freien Ende des Kopfteils (12) hin erstrecken.

14. Mundhygienegerät nach einem der vorhergehenden Ansprüche, **dadurch gekennzeichnet, dass** die Reinigungselemente (20, 22, 24, 25, 26, 28; 42; 54; 62; 70; 78; 84; 94; 100, 102, 104; 110, 112, 114; 116, 118; 124; 126; 128) an ihrem freien, der Grundfläche (18) abgewandtem Ende abgerundet und im Wesentlichen parallel zur Grundfläche (18) abgeflacht ausgebildet sind.

15. Mundhygienegerät nach Anspruch 14, **dadurch gekennzeichnet, dass** das abgerundete freie Ende nach Art eines Kreisbogens zwischen einer seitlichen Wangenfläche und einer stirnseitigen abgeflachten Kontaktfläche (36; 38) ausgebildet ist.

16. Mundhygienegerät nach einem der vorhergehenden Ansprüche 14 oder 15, **dadurch gekennzeichnet, dass** sich der Kreisbogen über einen Winkelbereich von 60° bis 120° erstreckt.

17. Mundhygienegerät nach einem der vorhergehenden Ansprüche 14 bis 16, **dadurch gekennzeichnet, dass** der Radius des Kreisbogens 10 Prozent bis 50 Prozent des Durchmessers (41) oder der Breite (32) des Reinigungselementes (20, 22, 24, 25, 26, 28; 42; 54; 62; 70; 78; 84; 94; 100, 102, 104; 110, 112, 114; 116, 118; 124; 126; 128) beträgt.

18. Mundhygienegerät nach einem der vorhergehenden Ansprüche 14 bis 17, **dadurch gekennzeichnet, dass** die Höhe des abgerundeten Bereichs zwischen 15 % und 40 % der Gesamthöhe (31) des Reinigungselements (20, 22, 24, 25, 26, 28; 42; 54; 62; 70; 78; 84; 94; 100, 102, 104; 110, 112, 114; 116, 118; 124; 126; 128) beträgt.

19. Mundhygienegerät nach einem der vorhergehenden Ansprüche 2 bis 18, **dadurch gekennzeichnet, dass** zumindest einige der Reinigungselemente (20, 22, 24, 25, 26, 28; 42; 54; 62; 70; 78; 84; 94; 100, 102, 104; 110, 112, 114; 116, 118; 124; 126; 128) in der Ebene parallel zur Grundfläche (18) eine kreisförmige oder elliptische Querschnittsfläche aufweisen.

20. Mundhygienegerät nach einem der vorhergehenden Ansprüche, **dadurch gekennzeichnet, dass** zumindest einige der Reinigungselemente (22, 24, 25, 26, 28; 84; 94) in der Ebene parallel zur Grundfläche (18) eine im Wesentlichen viereckige Querschnittsfläche aufweisen.

21. Mundhygienegerät nach einem der vorhergehenden Ansprüche, **dadurch gekennzeichnet, dass** zumindest einige der Reinigungselemente (84) eine in der Ebene parallel zur Grundfläche (18) rautenartig ausgebildete Querschnittsfläche aufweisen, wobei das Längenverhältnis der Rautendiagonalen (90, 92) zwischen 0,2 und 5 beträgt.

22. Mundhygienegerät nach einem der vorhergehenden Ansprüche, **dadurch gekennzeichnet, dass** zumindest eine der Seitenflächen (68; 72, 76) der Reinigungselemente (62; 70; 84) konvex und/oder konkav gewölbt ist.

23. Mundhygienegerät nach einem der vorhergehenden Ansprüche 20 bis 22, **dadurch gekennzeichnet, dass** Seitenbegrenzungen (76; 66) oder Seitenkanten (74; 88; 66; 58; 98, 99) der Reinigungselemente (22, 24, 25, 26, 28; 56; 62; 78; 84; 94) über nahezu die gesamte Höhe der Reinigungselemente (22, 24, 25, 26, 28; 56; 62; 78; 84; 94) abgerundet ausgebildet sind.

24. Mundhygienegerät nach einem der vorhergehenden Ansprüche, **dadurch gekennzeichnet, dass** Reinigungselemente (42) zur Flächennormalen der Grundfläche (18) unterschiedlich geneigte Seitenflächen (50, 48) aufweisen.

25. Mundhygienegerät nach einem der vorhergehenden Ansprüche, **dadurch gekennzeichnet, dass** zumindest einige der Reinigungselemente (54) kegelstumpfartig, insbesondere als halbierte, auf der Schnittfläche liegende Kegelstümpfe ausgebildet sind.

26. Mundhygienegerät nach einem der vorhergehenden Ansprüche, **dadurch gekennzeichnet, dass** Reinigungsnoppen (62) mit einer im Wesentlichen halbkugelartigen Grundgeometrie und mit einer im Wesentlichen schräg zur Grundfläche (18) verlaufenden konkav gewölbten Seitenfläche (68) vorgesehen sind.

27. Mundhygienegerät nach einem der vorhergehenden Ansprüche, **dadurch gekennzeichnet, dass** die Breite (32) oder der Durchmesser (41) der Reinigungselemente (20, 22, 24, 25, 26, 28; 42; 54; 62; 70; 78; 84; 94; 116, 118) geringer oder gleich ihrer Höhe (39) ist.

28. Mundhygienegerät nach einem der vorhergehenden Ansprüche, **dadurch gekennzeichnet, dass** die Breite (32) der Reinigungselemente (20, 22, 24, 25, 26, 28; 42; 54; 62; 70; 78; 84; 94; 116, 118) mindestens 0,5 mm, vorzugsweise zumindest 0,6 mm beträgt.

29. Mundhygienegerät nach einem der vorhergehenden Ansprüche, **dadurch gekennzeichnet, dass** die Höhe (39) der Reinigungselemente (20, 22, 24, 25, 26, 28; 42; 54; 62; 70; 78; 84; 94; 100, 102, 104; 110, 112, 114; 116, 118; 124; 126; 128) mindestens 0,6 mm, vorzugsweise zumindest 0,7 mm beträgt.

30. Mundhygienegerät nach einem der vorhergehenden Ansprüche, **dadurch gekennzeichnet, dass** die Grundfläche der Reinigungsnoppen (20, 42; 54; 62; 70; 78; 84; 94) zumindest 0,2 mm², vorzugsweise nicht weniger als 0,25 mm² beträgt.

31. Mundhygienegerät nach einem der vorhergehenden Ansprüche 2 bis 30, **dadurch gekennzeichnet, dass** die maximale Querschnittsfläche der im Schnitt kreisförmigen Reinigungsnoppen (20, 42; 54; 62; 70; 78; 84; 94) 0,8 mm² und für alle übrigen Reinigungsnoppen max. 4 mm², beträgt.

32. Mundhygienegerät nach einem der vorhergehenden Ansprüche, **dadurch gekennzeichnet, dass** in demjenigen Flächensegment der Grundfläche (18), in welchem die Reinigungsnoppen (20, 42; 54; 62; 70; 78; 84; 94) angeordnet sind, die Summe der Querschnittsfläche sämtlicher Reinigungsnoppen (20, 42; 54; 62; 70; 78; 84; 94) bis zu 75 % der Fläche dieses Flächensegments beträgt.

33. Mundhygienegerät nach einem der vorhergehenden Ansprüche, **dadurch gekennzeichnet, dass** zumindest einige der Reinigungsstäbchen (22, 24, 25, 26, 28; 42; 100, 102, 104; 110, 112, 114; 116, 118; 124; 126; 128) angewinkelt zur Längsrichtung (105) des Kopfteils (12) oder Griffteils (14) ausgerichtet sind.

34. Mundhygienegerät nach einem der vorhergehenden Ansprüche, **dadurch gekennzeichnet, dass** zumindest einige der Reinigungselemente (20, 22, 24, 25, 26, 28; 42; 54; 62; 70; 78; 84; 94; 100, 102, 104; 110, 112, 114; 116, 118; 124; 126; 128) spiegelsymmetrisch zur Längsachse (105) des Griffteils (14) oder des Kopfteils (12) ausgerichtet sind.

35. Mundhygienegerät nach einem der vorhergehenden Ansprüche, **dadurch gekennzeichnet, dass** die Reinigungsstäbchen (22, 24, 25, 26, 28; 42; 100, 102, 104; 110, 112, 114; 116, 118; 124; 126; 128) und/oder die Reinigungsnoppen (20, 42; 54; 62; 70; 78; 84; 94) punktsymmetrisch der Grundfläche (18) angeordnet sind.

36. Mundhygienegerät nach einem der vorhergehenden Ansprüche, **dadurch gekennzeichnet, dass** die Reinigungsstäbchen (100, 102, 104; 110, 112, 114; 124; 126; 128) zumindest bereichsweise eine in einem Winkelbereich von -30° bis +60° zur Flächennormalen der Grundfläche (18) geneigte Wangenflächen aufweisen.

37. Mundhygienegerät nach einem der vorhergehenden Ansprüche, **dadurch gekennzeichnet, dass** in demjenigen Flächensegment der Grundfläche (18), in welchem die Reinigungsstäbchen (22, 24, 25, 26, 28; 42; 100, 102, 104; 110, 112, 114; 116, 118; 124; 126; 128) angeordnet sind, die Summe der Querschnittsflächen der Reinigungsstäbchen (22, 24, 25, 26, 28; 42; 100, 102, 104; 110, 112, 114; 116, 118; 124; 126; 128) zwischen 5 Prozent und 50 Prozent der Fläche dieses Flächensegments beträgt.

38. Mundhygienegerät nach einem der vorhergehenden Ansprüche, **dadurch gekennzeichnet, dass** die minimale Breite des zumindest einen Kanals 0,8 mm und die maximale mittlere Breite des Kanals 1,5 mm beträgt.

39. Mundhygienegerät nach einem der vorhergehenden Ansprüche, **dadurch gekennzeichnet, dass** die Längserstreckung des zumindest einen Kanals mindestens 5 mm beträgt.

40. Mundhygienegerät nach einem der vorhergehenden Ansprüche, **dadurch gekennzeichnet, dass** in dem vom Griffteil (14) abgewandten Abschnitt der Grundfläche (18) zumindest ein geknickt verlaufendes Reinigungsstäbchen (100, 102, 104; 110, 112, 114; 124, 126, 128) mit zwei im Wesentlichen senkrecht zueinander ausgerichteten Schenkeln angeordnet ist.

41. Mundhygienegerät nach einem der vorhergehenden Ansprüche, **dadurch gekennzeichnet, dass** als Reinigungselemente in der Ebene der Grundfläche (18) gekrümmt verlaufende Reinigungsbögen (110, 112, 114; 124, 126, 128) vorgesehen sind.

42. Mundhygienegerät nach einem der Ansprüche 40 oder 41, **dadurch gekennzeichnet, dass** zumindest zwei entlang der Längsachse (105) des Griffteils (14) voneinander beabstandet angeordnete Reinigungsbögen (110, 112, 114; 124, 126, 128) oder geknickt verlaufende Reinigungsstäbchen (100, 102, 104) vorgesehen sind.

43. Mundhygienegerät nach einem der vorhergehenden Ansprüche 40 bis 42, **dadurch gekennzeichnet, dass** die zumindest zwei Reinigungsbögen (110, 112, 114; 124, 126, 128) oder Reinigungsstäbchen (100, 102, 104) unterschiedliche, zum Zentrum des Kopfteils (12) hin abnehmende Höhen aufweisen.

44. Mundhygienegerät nach einem der vorhergehenden Ansprüche, **dadurch gekennzeichnet, dass** die aus einem thermoplastischen Elastomer bestehenden Reinigungselemente (20, 22, 24, 25, 26, 28; 42; 54; 62; 70; 78; 84; 94; 100, 102, 104; 110, 112, 114; 116, 118; 124; 126; 128) eine Shore-Härte von 30 bis 70 A aufweisen.

45. Mundhygienegerät nach Anspruch 44, **dadurch gekennzeichnet, dass** die aus einem thermoplastischen Elastomer gebildete Grundfläche (18) nahtlos und einstückig zumindest in die Rückseite des Griffteils (14) übergeht.

46. Mundhygienegerät nach einem der vorhergehenden Ansprüche, **dadurch gekennzeichnet, dass** das Kopfteil (12) zumindest zwei Kunststoffmaterialien, insbesondere unterschiedlicher Elastizität oder Härte aufweist.

47. Mundhygienegerät nach einem der vorhergehenden Ansprüche, **dadurch gekennzeichnet, dass** das die Grundfläche (18) bildende Kunststoffmaterial das Kopfteil (12) seitlich umschließt.

48. Mundhygienegerät nach einem der vorhergehenden Ansprüche 46 oder 47, **dadurch gekennzeichnet, dass** das härtere Kunststoffmaterial im Bereich des Kopfteils zumindest einen Vorsprung (30) aufweist, welcher in die vom weicheren Kunststoff gebildete Grundfläche (18) flächenbündig eingebettet ist.

49. Mundhygienegerät nach Anspruch 48, **dadurch gekennzeichnet, dass** der in die Grundfläche (18) hineinragende Vorsprung (30) eine hellere Farbgebung und/oder einen geringeren optischen Absorptionsgrad als die Grundfläche (18) aufweist.

50. Mundhygienegerät nach einem der vorhergehenden Ansprüche, **dadurch gekennzeichnet, dass** im Zentrum der Grundfläche (18) zumindest ein Flächenabschnitt vorgesehen ist, der zur Aufnahme eines Reinigungsmediums ausgebildet ist.

51. Mundhygienegerät nach Anspruch 50, **dadurch gekennzeichnet, dass** der zur Aufnahme des Reinigungsmediums vorgesehene Flächenabschnitt eine Vertiefung für das Reinigungsmedium aufweist.

52. Mundhygienegerät nach einem der vorhergehenden Ansprüche, **dadurch gekennzeichnet, dass** die der Grundfläche (18) gegenüberliegende Vorderseite des Kopfteils (12) als ein mit Zahnbürstenborsten (16) bestückter Zahnbürstenkopf ausgebildet ist.

53. Mundhygienegerät nach einem der vorhergehenden Ansprüche, **dadurch gekennzeichnet, dass** die Summe aller etwa senkrecht relativ zur Grundfläche stehenden Flächen aller Reinigungselemente zwischen 50 mm² und 200 mm² beträgt.

## Claims

1. An oral hygiene device comprising a handle (14) and a head (12) on which is provided an essentially flat surface (18) on which are arranged projecting cleaning elements (20, 22, 24, 25, 26, 28; 42; 54; 62; 70; 78; 84; 94; 100, 102, 104; 110, 112, 114; 116, 118; 124; 126; 128) having at least two different base geometries, of which at least some are designed as elongated cleaning bars (22, 24, 25, 26, 28, 116, 118), wherein at least one channel is provided that runs transversely with respect to the longitudinal direction of cleaning bars (22, 24, 25, 26, 28, 116, 118) and out of spaces between cleaning elements (20, 22, 24, 25, 26, 28; 42; 54; 62; 70; 78; 84; 94; 100, 102, 104; 110, 112, 114; 116, 118; 124; 126; 128) that are arranged directly adjacent to each other, **characterized in that** cleaning nubs (20) having a nub-like design are provided as cleaning elements and **in that** at least one cleaning nub (20) is arranged in the extension of the length of the cleaning bars (22, 24, 25, 26, 28; 116, 118).

2. The oral hygiene device according to any of the preceding claims, **characterized in that** the at least one channel runs essentially perpendicular to the longitudinal direction of the relevant cleaning bars (22, 24, 25, 26, 28; 116, 118) adjacent to the channel.

3. The oral hygiene device according to any of the preceding claims **characterized in that** at least two channels are provided on the base surface (18) which run at least partially at an angle to each other.

4. The oral hygiene device according to any of the preceding claims, **characterized in that** the at least one channel runs in a straight line.

5. The oral hygiene device according to any of the preceding claims, **characterized in that** the at least one channel diverges or tapers.

6. The oral hygiene device according to any of the preceding claims 1 to 3 or 5, **characterized in that** the at least one channel runs in a curve.

7. The oral hygiene device according to any of the preceding claims, **characterized in that** at least two cleaning bars (22, 24, 25, 26, 28; 116, 118) are located with their longitudinal end section on opposite sides of the at least one channel.

8. The oral hygiene device according to any of the preceding claims, **characterized in that** the cleaning bars (22, 24, 25, 26, 28; 116, 118) located opposite the at least one channel are oriented with their longitudinal direction essentially perpendicular to the adjacent channel and are roughly parallel to each other.

9. The oral hygiene device according to any of the preceding claims, **characterized in that** the length of the cleaning bars (22, 24, 25, 26, 28; 116, 118) increases along the channel.

10. The oral hygiene device according to any of the preceding claims 1 to 3 or 5 to 9, **characterized in that** the curve radius of the channel decreases along the channel.

11. The oral hygiene device according to any of the preceding clams, **characterized in that** the cleaning nub (20) is arranged in an outer edge region and that cleaning bars (22, 24, 25, 26, 28; 116, 118) are arranged in the center area of the base surface (18).

12. The oral hygiene device according to any of the preceding claims, **characterized in that** the cleaning bars (116, 118) are arranged in the manner of fish scales, spaced apart from each other alternating in different directions along the longitudinal axis (105) of the head (12), and arranged partially overlapping in a direction transverse to the head (12).

13. The oral hygiene device according to any of the preceding claims **characterized in that** the cleaning bars (22, 24, 25, 26, 28; 116, 118) extend from the edge of the head (12) towards the center and towards the free end of the head (12).

14. The oral hygiene device according to any of the preceding claims, **characterized in that** the cleaning elements (20, 22, 24, 25, 26, 28; 42; 54; 62; 70; 78; 84; 94; 100, 102, 104; 110, 112, 114; 116, 118; 124; 126; 128) are rounded at their free end which faces away from the base surface (18) and are flattened essentially parallel to the base surface (18).

15. The oral hygiene device according to claim 14, **characterized in that** the rounded free end is formed as an arc between a lateral cheek surface and a contact surface flattened on the face (36; 38).

16. The oral hygiene device according to either of the preceding claims 14 or 15 **characterized in that** the arc extends over a range of between 60° to 120°.

17. The oral hygiene device according to either of the preceding claims 14 to 16, **characterized in that** the radius of the arc amounts to 10 percent to 50 percent of the diameter (41) of the width (32) of the cleaning element (20, 22, 24, 25, 26, 28; 42; 54; 62; 70; 78; 84; 94; 100, 102, 104; 110, 112, 114; 116, 118; 124; 126; 128).

18. The oral hygiene device according to either of the preceding claims 14 to 17, **characterized in that** the height of the rounded area amounts to between 15% and 40% of the total height (31) of the cleaning element (20, 22, 24, 25, 26, 28; 42; 54; 62; 70; 78; 84; 94; 100, 102, 104; 110, 112, 114; 116, 118; 124; 126; 128).

19. The oral hygiene device according to any of the preceding claims 2 to 18, **characterized in that** at least some of the cleaning elements (20, 22, 24, 25, 26, 28; 42; 54; 62; 70; 78; 84; 94; 100, 102, 104; 110, 112, 114; 116, 118; 124; 126; 128) have a circular or an elliptical cross-section parallel to the plane of the base surface (18).

20. The oral hygiene device according to any of the preceding claims, **characterized in that** at least some of the cleaning elements (22, 24, 25, 26, 28; 84; 94) have an essentially four-sided cross-sectional area in the plane parallel to the base surface (18).

21. The oral hygiene device according to any of the preceding claims, **characterized in that** at least some of the cleaning elements (84) have a diamond-shaped cross-sectional area in the plane parallel to the base surface (18), wherein the aspect ratio of the diamond shape's diagonals (90, 92) ranges between 0.2 and 5.

22. The oral hygiene device according to any of the preceding claims, **characterized in that** at least one of the lateral surfaces (68; 72, 76) of the cleaning elements (62; 70; 84) is convexly or concavely formed.

23. The oral hygiene device according to any of the preceding claims 20 to 22, **characterized in that** lateral boundaries (76; 66) or lateral edges (74; 88; 66; 58; 98, 99) of the cleaning elements (22, 24, 25, 26, 28; 56; 62; 78; 84; 94) are rounded over almost the whole height of the cleaning elements (22, 24, 25, 26, 28; 56; 62; 78; 84; 94).

24. The oral hygiene device according to any of the preceding claims, **characterized in that** the cleaning elements (42) have lateral surfaces (50, 48) which are varyingly inclined relative to the surface normal of the base surface (18).

25. The oral hygiene device according to any of the preceding claims, **characterized in that** at least some of the cleaning elements (54) are formed as truncated cones, in particular as half-cones situated on the cut face.

26. The oral hygiene device according to any of the preceding claims, **characterized in that** cleaning nubs (62) are provided which have an essentially hemispherical base geometry and a concavely formed lateral surface (68) which runs essentially diagonally to the base surface (18).

27. The oral hygiene device according to any of the preceding claims, **characterized in that** the width (32) or the diameter (41) of the cleaning elements (20, 22, 24, 25, 26, 28; 42; 54; 62; 70; 78; 84; 94; 116, 118) is less than or equal to their height (39).

28. The oral hygiene device according to any of the preceding claims, **characterized in that** the width (32) of the cleaning elements (20, 22, 24, 25, 26, 28; 42; 54; 62; 70; 78; 84; 94; 116, 118) is at least 0.5 mm, preferably at least 0.6 mm.

29. The oral hygiene device according to any of the preceding claims, **characterized in that** the height (39) of the cleaning elements (20, 22, 24, 25, 26, 28; 42; 54; 62; 70; 78; 84; 94; 100, 102, 104; 110, 112, 114; 116, 118; 124; 126; 128) is at least 0.6 mm, preferably at least 0.7 mm.

30. The oral hygiene device according to any of the preceding claims, **characterized in that** the base surface of the cleaning nubs (20, 42; 54; 62; 70; 78; 84; 94) is at least 0.2 mm.², preferably not less than 0.25 mm².

31. The oral hygiene device according to any of the preceding claims 2 to 30, **characterized in that** the maximum cross-sectional area of the cleaning nubs (20, 42; 54; 62; 70; 78; 84; 94), which are circular in their cross-section, is 0.8 mm² and a maximum of 4 mm² for all of the other cleaning nubs.

32. The oral hygiene device according to any of the preceding claims, **characterized in that**, in the surface segment of the base surface (18) in which the cleaning nubs (20, 42; 54; 62; 70; 78; 84; 94) are arranged, the sum of the cross-sectional area of all of the cleaning nubs (20, 42; 54; 62; 70; 78; 84; 94) equals up to 75% of the surface area of this surface segment.

33. The oral hygiene device according to any of the preceding claims, **characterized in that** at least some of the cleaning bars (22, 24, 25, 26, 28; 42; 100, 102, 104; 110, 112, 114; 116, 118; 124; 126; 128) are angled with respect to the longitudinal direction (105) of the head (12) or the handle (14).

34. The oral hygiene device according to any of the preceding claims, **characterized in that** at least some of the cleaning elements (20, 22, 24, 25, 26, 28; 42; 54; 62; 70; 78; 84; 94; 100, 102, 104; 110, 112, 114; 116, 118; 124; 126; 128) are arranged in mirror symmetry with respect to the longitudinal axis (105) of the handle (14) or of the head (12).

35. The oral hygiene device according to any of the preceding claims, **characterized in that** the cleaning bars (22, 24, 25, 26, 28; 42; 100, 102, 104; 110, 112, 114; 116, 118; 124; 126; 128) and /or the cleaning nubs (20, 42; 54; 62; 70; 78; 84; 94) are arranged in point symmetry with respect to the base surface (18).

36. The oral hygiene device according to any of the preceding claims, **characterized in that** the cleaning bars (100, 102, 104; 110, 112, 114; 124; 126; 128) have cheek surfaces at least partially inclined at an angle range of from -30° to +60° to the surface normal of the base surface (18).

37. The oral hygiene device according to any of the preceding claims, **characterized in that**, in the surface segment of the base surface (18) in which the cleaning bars (22, 24, 25, 26, 28; 42; 100, 102, 104; 110, 112, 114; 116, 118; 124; 126; 128) are arranged, the sum of the cross-sectional area of the cleaning bars (22, 24, 25, 26, 28; 42; 100, 102, 104; 110, 112, 114; 116, 118; 124; 126; 128) is between 5 percent and 50 percent of the surface area of this surface segment.

38. The oral hygiene device according to any of the preceding claims, **characterized in that** the minimum width of the at least one channel is 0.8 mm and the maximum mean width of the channel is 1.5 mm.

39. The oral hygiene device according to any of the preceding claims, **characterized in that** the length of the at least one channel is at least 5 mm.

40. The oral hygiene device according to any of the preceding claims, **characterized in that**, in the section of the base surface (18) that faces away from the handle (14), at least one of the cleaning bars (100, 102, 104; 110, 112, 114; 124, 126, 128) having a bent course is arranged with two legs oriented essentially perpendicular to one another.

41. The oral hygiene device according to any of the preceding claims, **characterized in that** cleaning arcs (110, 112, 114; 124, 126, 128) are provided as cleaning elements which run in a curve in the plane of the base surface (18).

42. The oral hygiene device according to either of claims 40 or 41, **characterized in that** at least two cleaning arcs (110, 112, 114; 124, 126, 128) or cleaning bars running with a bend (100, 102, 104) are provided along the longitudinal axis (105) of the handle (14) at a distance to one another.

43. The oral hygiene device according to any of the preceding claims 40 to 42, **characterized in that** the at least two cleaning arcs (110, 112, 114; 124, 126, 128) or cleaning bars (100, 102, 104) have different heights decreasing towards the center of the head (12).

44. The oral hygiene device according to any of the preceding claims, **characterized in that** the thermoplastic elastomer cleaning elements (20, 22, 24, 25, 26, 28; 42; 54; 62; 70; 78; 84; 94; 100, 102, 104; 110, 112, 114; 116, 118; 124; 126; 128) have a Shore hardness of from 30 to 70 A.

45. The oral hygiene device according to claim 44, **characterized in that** the thermoplastic elastomer base surface (18) merges seamlessly and in one piece into at least the back of the handle (14).

46. The oral hygiene device according to any of the preceding claims, **characterized in that** the head (12) has at least two plastic materials, in particular of differing elasticity or hardness.

47. The oral hygiene device according to any of the preceding claims, **characterized in that** the plastic material forming the base surface (18) laterally encompasses the head (12).

48. The oral hygiene device according to either of the preceding claims 46 or 47, **characterized in that** the harder plastic material in the area of the head has at least one projection (30), which is embedded in a flush manner in the base surface (18) made of a softer plastic material.

49. The oral hygiene device according to claim 48, **characterized in that** the projection (30) protruding into the base surface (18) has a brighter coloration and/or a lower degree of optical absorption than the base surface (18).

50. The oral hygiene device according to any of the preceding claims, **characterized in that** at least one surface section is provided in the center of the base surface (18) which is designed to hold a cleaning medium.

51. The oral hygiene device according to claim 50, **characterized in that** the surface section designed to hold the cleaning medium has a recess for the cleaning medium.

52. The oral hygiene device according to any of the preceding claims, **characterized in that** the front side of the head (12) opposing the base surface (18) is designed as a toothbrush head fitted with toothbrush bristles (16).

53. The oral hygiene device according to any of the preceding claims, **characterized in that** the sum of all of the surface areas of the cleaning elements that are roughly perpendicular to the base surface is between 50 mm² and 200 mm².

## Revendications

1. Appareil pour l'hygiène buccale doté d'une partie manche (14) et d'une partie tête (12), sur laquelle est prévue une surface essentiellement plane (18), sur laquelle sont disposés des éléments de nettoyage en saillie (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128) avec au moins deux géométries de base différentes, dont au moins certains sont formés comme des petites barres de nettoyage allongées (22, 24, 25, 26, 28, 116, 118), au moins un canal étant prévu, s'étendant transversalement par rapport à la direction longitudinale des petites barres de nettoyage (22, 24, 25, 26, 28, 116, 118) et formé comme des interstices entre des éléments de nettoyage (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128) disposés immédiatement adjacents, **caractérisé en ce que** des éléments en saillie pour le nettoyage (20) qui ont la forme de boutons sont prévues comme éléments de nettoyage et **en ce qu'**au moins un élément en saillie pour le nettoyage (20) est disposé dans la prolongation de l'étendue longitudinale des petites barres de nettoyage (22, 24, 25, 26, 28 ; 116, 118).

2. Appareil pour l'hygiène buccale selon l'une des revendications précédentes, **caractérisé en ce que** l'au moins un canal s'étend essentiellement perpendiculaire à la direction longitudinale des petites barres de nettoyage (22, 24, 25, 26, 28 ; 116, 118) respectives adjacentes au canal.

3. Appareil pour l'hygiène buccale selon l'une des revendications précédentes, **caractérisé en ce qu'**au moins deux canaux, s'étendant au moins partiellement en biais l'un par rapport à l'autre, sont prévus sur la surface de base (18).

4. Appareil pour l'hygiène buccale selon l'une des revendications précédentes, **caractérisé en ce que** l'au moins un canal s'étend de manière linéaire.

5. Appareil pour l'hygiène buccale selon l'une des revendications précédentes, **caractérisé en ce que** l'au moins un canal s'étend de façon à s'écarter ou se rétrécir.

6. Appareil pour l'hygiène buccale selon l'une des revendications précédentes 1 à 3 ou 5, **caractérisé en ce que** l'au moins un canal s'étend de façon courbe.

7. Appareil pour l'hygiène buccale selon l'une des revendications précédentes, **caractérisé en ce qu'**au moins deux petites barres de nettoyage (22, 24, 25, 26, 28 ; 116, 118) viennent reposer avec leur section d'extrémité côté longitudinal sur des côtés opposés dudit au moins un canal.

8. Appareil pour l'hygiène buccale selon l'une des revendications précédentes, **caractérisé en ce que** les petites barres de nettoyage (22, 24, 25, 26, 28 ; 116, 118) venant reposer à l'opposé sur au moins un canal sont orientées dans leur direction longitudinale de manière essentiellement perpendiculaire au canal adjacent et approximativement parallèle l'un par rapport à l'autre.

9. Appareil pour l'hygiène buccale selon l'une des revendications précédentes, **caractérisé en ce que** l'étendue longitudinale des petites barres de nettoyage (22, 24, 25, 26, 28 ; 116, 118) augmente le long du canal.

10. Appareil pour l'hygiène buccale selon l'une des revendications précédentes 1 à 3 ou 5 à 9, **caractérisé en ce que** le rayon de courbure du canal diminue le long du canal.

11. Appareil pour l'hygiène buccale selon l'une des revendications précédentes, **caractérisé en ce que** les éléments en saillie pour le nettoyage (20) se trouvent dans une zone de bord extérieure et **en ce que** les petites barres de nettoyage (22, 24, 25, 26, 28 ; 116, 118) sont agencées dans une zone centrale de la surface de base (18).

12. Appareil pour l'hygiène buccale selon l'une des revendications précédentes, **caractérisé en ce que** les petites barres de nettoyage (116, 118) sont écartées les unes des autres sous la forme d'un motif de chevrons, de manière alternée, dans des directions différentes le long de l'axe longitudinal (105) de la partie tête (12) et sont agencées dans la direction transversale de la partie tête (12) de façon à se chevaucher par endroits.

13. Appareil pour l'hygiène buccale selon l'une des revendications précédentes, **caractérisé en ce que** les petites barres de nettoyage (22, 24, 25, 26, 28 ; 116, 118) s'étendent depuis le bord de la partie tête (12) jusqu'au centre et jusqu'aux extrémités libres de la partie tête (12).

14. Appareil pour l'hygiène buccale selon l'une des revendications précédentes, **caractérisé en ce que** les éléments de nettoyage (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128) sont arrondis au niveau de leur extrémité libre opposée à la surface de base (18) et sont formés aplatis de façon essentiellement parallèle à la surface de base (18).

15. Appareil pour l'hygiène buccale selon la revendication 14, **caractérisé en ce que** l'extrémité libre arrondie est formée comme un arc de cercle entre une surface de joue latérale et une surface de contact aplatie côté avant (36 ; 38).

16. Appareil pour l'hygiène buccale selon l'une des revendications précédentes 14 ou 15, **caractérisé en ce que** l'arc de cercle s'étend sur une plage angulaire de 60 ° à 120 °.

17. Appareil pour l'hygiène buccale selon l'une des revendications précédentes 14 à 16, **caractérisé en ce que** le rayon de l'arc de cercle représente de 10 pour cent à 50 pour cent du diamètre (41) ou de la largeur (32) des éléments de nettoyage (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128).

18. Appareil pour l'hygiène buccale selon l'une des revendications précédentes 14 à 17, **caractérisé en ce que** la hauteur de la zone arrondie est comprise entre 15 % et 40 % de la hauteur totale (31) des éléments de nettoyage (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128).

19. Appareil pour l'hygiène buccale selon l'une des revendications précédentes 2 à 18, **caractérisé en ce qu'**au moins certains des éléments de nettoyage (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128) présentent, dans le plan parallèle à la surface de base (18), une surface en section transversale circulaire ou elliptique.

20. Appareil pour l'hygiène buccale selon l'une des revendications précédentes, **caractérisé en ce qu'**au moins certains des éléments de nettoyage (22, 24, 25, 26, 28 ; 84 ; 94) présentent, dans le plan parallèle à la surface de base (18), une surface en section transversale essentiellement carrée.

21. Appareil pour l'hygiène buccale selon l'une des revendications précédentes, **caractérisé en ce qu'**au moins certains des éléments de nettoyage (84) présentent une surface en section transversale formée en losange dans le plan parallèle à la surface de base (18), dans lequel le rapport de longueur des diagonales du losange (90, 92) est compris entre 0,2 et 5.

22. Appareil pour l'hygiène buccale selon l'une des revendications précédentes, **caractérisé en ce qu'**au moins une des surfaces latérales (68 ; 72, 76) des éléments de nettoyage (62 ; 70 ; 84) est bombée de manière convexe et/ou concave.

23. Appareil pour l'hygiène buccale selon l'une des revendications précédentes 20 à 22, **caractérisé en ce que** des limites latérales (76 ; 66) ou arêtes latérales (74 ; 88 ; 66 ; 58 ; 98, 99) des éléments de nettoyage (22, 24, 25, 26, 28 ; 56 ; 62 ; 78 ; 84 ; 94) sur presque toute la hauteur des éléments de nettoyage (22, 24, 25, 26, 28 ; 56 ; 62 ; 78 ; 84 ; 94) sont formées arrondies.

24. Appareil pour l'hygiène buccale selon l'une des revendications précédentes, **caractérisé en ce que** les éléments de nettoyage (42) présentent des surfaces latérales (50, 48) inclinées différemment par rapport à la normale de la surface de base (18).

25. Appareil pour l'hygiène buccale selon l'une des revendications précédentes, **caractérisé en ce qu'**au moins quelques-uns des éléments de nettoyage (54) sont formés tronconiques, notamment en forme de cônes tronqués réduits de moitié reposant sur la surface d'intersection.

26. Appareil pour l'hygiène buccale selon l'une des revendications précédentes, **caractérisé en ce que** les éléments en saillie pour le nettoyage (62) sont dotées d'une géométrie de base essentiellement hémisphérique et d'une surface latérale (68) bombée concave s'étendant essentiellement inclinée par rapport à la surface de base (18).

27. Appareil pour l'hygiène buccale selon l'une des revendications précédentes, **caractérisé en ce que** la largeur (32) ou le diamètre (41) des éléments de nettoyage (20, 22, 24, 25, 26, 28 ; 42 ; 54 ; 62 ; 70 ; 78 ; 84 ; 94 ; 116, 118) est inférieur(e) ou égal(e) à leur hauteur (39).

28. Appareil pour l'hygiène buccale selon l'une des revendications précédentes, **caractérisé en ce que** la largeur (32) des éléments de nettoyage (20, 22, 24, 25, 26, 28 ; 42 ; 54 ; 62 ; 70 ; 78 ; 84 ; 94 ; 116, 118) est d'au moins 0,5 mm, de préférence d'au moins 0,6 mm.

29. Appareil pour l'hygiène buccale selon l'une des revendications précédentes, **caractérisé en ce que** la hauteur (39) des éléments de nettoyage (20, 22, 24, 25, 26, 28; 42; 54; 62; 70; 78; 84; 94; 100, 102,104; 110, 112, 114; 116, 118; 124 ; 126 ; 128) est d'au moins 0,6 mm, de préférence d'au moins 0,7 mm.

30. Appareil pour l'hygiène buccale selon l'une des revendications précédentes, **caractérisé en ce que** la surface de base des éléments en saillie pour le nettoyage (20, 42 ; 54 ; 62 ; 70 ; 78 ; 84 ; 94) est d'au moins 0,2 mm², de préférence non inférieure à 0,25 mm².

31. Appareil pour l'hygiène buccale selon l'une des revendications précédentes 2 à 30, **caractérisé en ce que** la surface en section transversale maximale des éléments en saillie pour le nettoyage (20, 42 ; 54 ; 62 ; 70 ; 78 ; 84 ; 94) de forme circulaire en coupe est de 0,8 mm² et pour toutes les autres éléments en saillie pour le nettoyage est de max. 4 mm².

32. Appareil pour l'hygiène buccale selon l'une des revendications précédentes, **caractérisé en ce que**, dans le segment de surface de la surface de base (18), dans lequel les éléments en saillie pour le nettoyage (20, 42 ; 54 ; 62 ; 70 ; 78 ; 84 ; 94) sont disposés, la somme des surfaces en section transversale de tous les éléments en saillie pour le nettoyage (20, 42 ; 54 ; 62 ; 70 ; 78 ; 84 ; 94) représente jusqu'à 75 % de la surface de ce segment de surface.

33. Appareil pour l'hygiène buccale selon l'une des revendications précédentes, **caractérisé en ce qu'**au moins quelques-unes des petites barres de nettoyage (22, 24, 25, 26, 28 ; 42 ; 100, 102, 104 ; 110, 112, 114 ; 116, 118 ; 124 ; 126 ; 128) sont orientées de manière angulaire par rapport à la direction longitudinale (105) de la partie tête (12) ou de la partie manche (14).

34. Appareil pour l'hygiène buccale selon l'une des revendications précédentes, caractérisé en qu'au moins quelques-uns des éléments de nettoyage (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128) sont orientés de manière symétrique par réflexion par rapport à l'axe longitudinal (105) de la partie manche (14) ou de la partie tête (12).

35. Appareil pour l'hygiène buccale selon l'une des revendications précédentes, **caractérisé en ce que** les petites barres de nettoyage (22, 24, 25, 26, 28 ; 42 ; 100, 102, 104 ; 110, 112, 114 ; 116, 118 ; 124 ; 126 ; 128) et/ou les éléments en saillie pour le nettoyage (20, 42 ; 54 ; 62 ; 70 ; 78 ; 84 ; 94) sont orientés de manière symétrique par point par rapport à la surface de base (18).

36. Appareil pour l'hygiène buccale selon l'une des revendications précédentes, **caractérisé en ce que** les petites barres de nettoyage (100, 102, 104 ; 110, 112, 114 ; 124 ; 126 ; 128) présentent, au moins par endroits, une surface de joue inclinée selon une plage angulaire de -30 ° à +60 ° par rapport à la normale de la surface de base (18).

37. Appareil pour l'hygiène buccale selon l'une des revendications précédentes, **caractérisé en ce que**, dans le segment de surface de la surface de base (18), dans lequel les petites barres de nettoyage (22, 24, 25, 26, 28 ; 42 ; 100, 102, 104 ; 110, 112, 114 ; 116, 118 ; 124 ; 126 ; 128) sont agencées, la somme des surfaces en section transversale des barres de nettoyage (22, 24, 25, 26, 28 ; 42 ; 100, 102, 104 ; 110, 112, 114 ; 116, 118 ; 124 ; 126 ; 128) est comprise entre 5 pour cent et 50 pour cent de la surface de ce segment de surface.

38. Appareil pour l'hygiène buccale selon l'une des revendications précédentes, **caractérisé en ce que** la largeur minimale de l'au moins un canal est de 0,8 mm et la largeur intermédiaire maximale du canal est de 1,5 mm.

39. Appareil pour l'hygiène buccale selon l'une des revendications précédentes, **caractérisé en ce que** l'étendue longitudinale de l'au moins un canal est d'au moins 5 mm.

40. Appareil pour l'hygiène buccale selon l'une des revendications précédentes, **caractérisé en ce que**, dans la section à l'opposé de la partie manche (14) de la surface de base (18), au moins une petite tige de nettoyage (100, 102, 104 ; 110, 112, 114 ; 124, 126, 128) s'étendant de façon coudée est disposée avec deux branches orientées essentiellement perpendiculairement l'une par rapport à l'autre.

41. Appareil pour l'hygiène buccale selon l'une des revendications précédentes, **caractérisé en ce que**, en tant qu'éléments de nettoyage dans le plan de la surface de base (18), sont prévus des éléments de nettoyage en voûte (110, 112, 114 ; 124, 126, 128) s'étendant courbés.

42. Appareil pour l'hygiène buccale selon l'une des revendications 40 ou 41, **caractérisé en ce qu'**au moins deux éléments de nettoyage en voûte (110, 112, 114 ; 124, 126, 128) agencés espacés l'un de l'autre le long de l'axe longitudinal (105) de la partie manche (14) ou petites barres de nettoyage (100, 102, 104) s'étendant coudées, sont prévues.

43. Appareil pour l'hygiène buccale selon l'une des revendications précédentes 40 à 42, **caractérisé en ce que** les au moins deux éléments de nettoyage en voûte (110, 112, 114 ; 124, 126, 128) ou petites barres de nettoyage (100, 102, 104) présentent des hauteurs différentes, décroissantes jusqu'au centre de la partie tête (12).

44. Appareil pour l'hygiène buccale selon l'une des revendications précédentes, **caractérisé en ce que** les éléments de nettoyage (20, 22, 24, 25, 26, 28 ; 42 ; 54 ; 62 ; 70 ; 78 ; 84 ; 94 ; 100, 102, 104 ; 110, 112, 114 ; 116, 118 ; 124 ; 126 ; 128) composés d'un élastomère thermoplastique présentent une dureté Shore de 30 à 70 A.

45. Appareil pour l'hygiène buccale selon la revendication 44, **caractérisé en ce que** la surface de base (18) formée d'un élastomère thermoplastique se joint sans discontinuité et d'un seul tenant au moins au côté arrière de la partie manche (14).

46. Appareil pour l'hygiène buccale selon l'une des revendications précédentes, **caractérisé en ce que** la partie tête (12) présente au moins deux matériaux de plastique, notamment de différente élasticité ou dureté.

47. Appareil pour l'hygiène buccale selon l'une des revendications précédentes, **caractérisé en ce que** le matériau plastique formant la surface de base (18) entoure latéralement la partie tête (12).

48. Appareil pour l'hygiène buccale selon l'une des revendications précédentes 46 ou 47, **caractérisé en ce que** le matériau plastique plus dur dans la zone de la partie tête présente au moins une saillie (30), qui est intégrée à fleur dans la surface de base (18) formée d'un plastique plus souple.

49. Appareil pour l'hygiène buccale selon la revendication 48, **caractérisé en ce que** la saillie (30) s'étendant à l'intérieur de la surface de base (18) présente une coloration plus claire et/ou un degré d'absorption optique plus faible que la surface de base (18).

50. Appareil pour l'hygiène buccale selon l'une des revendications précédentes, **caractérisé en ce que**, au centre de la surface de base (18), au moins une section de surface est prévue, qui est formée pour la réception d'un agent de nettoyage.

51. Appareil pour l'hygiène buccale selon la revendication 50, **caractérisé en ce que** la section de surface prévue pour la réception de l'agent de nettoyage présente un renfoncement pour l'agent de nettoyage.

52. Appareil pour l'hygiène buccale selon l'une des revendications précédentes, **caractérisé en ce que** le côté avant de la partie tête (12) à l'opposé de la surface de base (18) est formé comme une tête de brosse à dents équipée de poils de brosse à dents(16).

53. Appareil pour l'hygiène buccale selon l'une des revendications précédentes, **caractérisé en ce que** la somme de toutes les surfaces étant approximativement perpendiculaires par rapport à la surface de base de tous les éléments de nettoyage est de 50 mm² et 200 mm².
